# EUROPEAN PATENT SPECIFICATION

(11) **EP 4 192 320 B1**
(45) Date of publication and mention of the grant of the patent: **22.04.2026**
(21) Application number: 21763261.1
(22) Date of filing: 06.08.2021
(51) Int. Cl.: A61B 1/00

(54) **DEVICE FOR COUPLING COHERENT LIGHT INTO AN ENDOSCOPIC SYSTEM**
VORRICHTUNG ZUR EINKOPPLUNG VON KOHÄRENTEM LICHT IN EIN ENDOSKOPSYSTEM
DISPOSITIF DE COUPLAGE DE LUMIÈRE COHÉRENTE DANS UN SYSTÈME ENDOSCOPIQUE

(30) Priority: 07.08.2020 NL 2026240
(43) Date of publication of application: 14.06.2023
(73) Proprietor: LIMIS Development B.V., 8934 AD Leeuwarden (NL)
(72) Inventor: CALON, Joost Emiel Marcel, 8401 DK Gorredijk (NL); DE GRAAF, Joris Sebastiaan, 5223 LH 's-Hertogenbosch (NL)
(74) Representative: De Vries & Metman
(86) International application number: PCT/EP2021/072041
(87) International publication number: WO 2022/029308

(56) References cited:
- EP-A1- 1 324 051
- JP-A- 2007 209 449
- JP-A- 2015 130 968
- US-A1- 2003 191 368

## Description

### Field of the invention

The disclosure relates to coupling coherent light into a light delivery system of an endoscopic system and in particular to devices, systems and methods for coupling coherent light into a light delivery system of an endoscopic system and to computer program products using such methods.

### Background

Endoscopic systems are used to view or image regions within a human or animal body, typically during medical procedures, e.g., surgery or diagnostics. For the scope of this disclosure, endoscopic systems may include systems for internal inspection of both naturally accessible body parts and cavities with artificially created openings, e.g., laparoscopic systems, arthroscopic systems, bronchoscopic systems, neuroendoscopic systems, et cetera. Endoscopic systems typically include an endoscope comprising a light delivery system and an image guide, a white light source, and an imaging device such as a camera head. Typically, an endoscope includes a rigid or flexible elongated insertion tube equipped with a first light guide, e.g., a fibre bundle, to illuminate a target, and a second light guide, e.g., a rod lens, to transfer an image of the target to the imaging system. Other endoscopes may have an imaging sensor at or near the tip, so-called chip-on-the-tip systems. The endoscope is connectable to the (external) light source and a screen using cables. The white light illumination provides a surgeon or endoscopist with an easy to interpret image.

Many modern medical imaging techniques require coherent light, e.g., laser light. For example, laser speckle contrast imaging (LSCI), sometimes referred to as laser speckle contrast analysis (LASCA), uses coherent light to provide a fast, full-field, cheap, and relatively simple *in vivo* imaging method for determining two-dimensional perfusion maps of living biological tissue. Perfusion can be an indicator for tissue viability and thus may provide valuable information during diagnostics and surgery. As a further example, fluorescent imaging, e.g., indocyanine green (ICG) based perfusion imaging, may require coherent light to excite fluorescent markers.

It is therefore desirable to combine the advantages of white light imaging and coherent light imaging using a single endoscope.

WO2015/176294A1 describes a laparoscopic system for imaging subsurface blood flow of tissue based on laser speckle contrast imaging. The system comprises a white light source, a laparoscope with a light guide for transmitting the white light to a field of view, a laser light source, and an optical fibre to transmit the laser light to the field of view. The optical fibre may be inserted in a body of a patient via a separate incision, or the optical fibre may be integrated as a separate channel into or onto a shaft of the laparoscope, which requires replacing or at least substantially altering the laparoscope.

US2016/0022126A1 describes an endoscopic system with a single light source which is capable of providing white light and infrared laser light for exciting fluorescent markers. However, using this system would result in replacing the entire endoscopic system, or at the very least the entire light source. This is relatively expensive.

EP 1 324 051 A1 discloses an endoscopic blood flow measurement system with an external laser transmitter and white light source connected to a basic switching device positioned directly at the endoscope entrance port. The system uses separate fiber connections for laser and white light, wherein standard fiber guides cause mode degradation for laser applications.

US 2003/191368 describes a multi-spectral endoscopic imaging system with integrated UV laser and white light sources controlled by computer-operated shutters. The system maintains completely separate optical paths, delivering UV light through dedicated quartz fibers while white light travels through standard illumination ports, with sequential switching between the two light sources.

Hence, from the above, it follows that there is a need in the art for a system to combine the advantages of coherent light imaging with an existing endoscopic system.

### Summary

It is an objective of the embodiments in this disclosure to reduce or eliminate at least one of the drawbacks known in the prior art.

In a first aspect, the invention relates to a coupling device for coupling coherent light and incoherent light into a first light guide of a light delivery system of an endoscopic system, the endoscopic system comprising an incoherent light source for generating the incoherent light, e.g., white light. The coupling device comprises an optical input comprising an optical input connector for connecting, preferably releasably connecting, the coupling device to the incoherent light source via a second light guide, the optical input being configured for receiving the incoherent light from the endoscopic system; a first light source for generating coherent light of a first wavelength; and an optical output comprising an optical output connector for releasably connecting the coupling device to an endoscope via the first light guide, the optical output being configured for providing the incoherent light and the coherent light of the first wavelength to the first light guide. The coupling device is configurable in a first state and a second state, in which first state, the coupling device comprises an unobstructed incoherent light beam path for propagating the incoherent light from the optical input to the optical output; and in which second state, the coupling device comprises an unobstructed coherent light beam path for propagating the coherent light from the coherent light source or from the coherent light input to the optical output. The coupling device further comprises an optical coupler for alternately injecting the incoherent light and the coherent light of the first wavelength into the first light guide, wherein the coherent light and the incoherent light have a shared optical path at the optical output.

The optical output comprises a single output channel, possibly comprising a plurality of fibres, for providing the incoherent light and/or the coherent light to the first light guide of the light delivery system of the endoscopic system. Thus, the coherent light and the incoherent light have a shared optical path at the optical output of the coupling device and consequently at the entrance of the first light guide. The first state and the second state may be distinct states, resulting in a time-division manner of sharing the optical path.

In some cases, a cable comprising the first light guide may comprise additional channels. The coupling device may comprise additional outputs (i.e., in addition to the optical output) for providing input to such additional channels.

As used herein, light being provided to a light guide means that the light is injected into a light entrance of the light guide. When light is provided to an optical output, it is understood that the light is also provided to a light guide connected to the optical output, when present.

The incoherent light may be white light or coloured light, e.g., blue light. The incoherent light source may also comprise a plurality of coherent light sources providing coherent light simultaneously or alternately, e.g., in a time-division manner. The endoscopic system may be any system for internal visual inspection of objects, preferably human or animal bodies, preferably living bodies. Internal visual inspection may relate both to naturally accessible body parts such as the bladder, intestines, or bronchia, and to cavities with artificially created openings such as the abdominal cavity or with neurosurgery. Thus, the endoscopic system may be, e.g., a laparoscopic system, an arthroscopic system, a bronchoscopic system, a neuroendoscopic system, et cetera. The endoscopic system may further comprise surgical instruments, e.g., a robotic surgery system.

By connecting the coupling device between the incoherent light source of the endoscopic system and an endoscope proper, coherent light may be guided into first light guide of the light delivery system of the endoscope, thus allowing for additional imaging capabilities, such as LSCI and/or fluorescence imaging, e.g., indocyanine green (ICG) imaging for perfusion imaging or angiography. Other fluorescent markers may be used for other applications. For example, fluorescence imaging may also be used to detect tumour tissue. In general, different markers are activated by light of different wavelengths.

By using a separate device, there is no need to replace the light source or even the entire endoscopic system, or to disconnect and reconnect the first light guide of the light delivery system each time a user wishes to switch between an incoherent light source and a coherent light source. Thus, it is a relatively economical, flexible, and easy to operate option.

The coupling device is connectable to the first light guide of light delivery system, rather than directly to a light input of the endoscope proper. This way, the coupling device can be placed outside a sterile zone in e.g., an operation theatre. This way, the device does not need to be sterilised before each use, increasing ease of use and reducing both production costs and operating costs. By contrast, a device that would be attached directly to an endoscope would need to be compatible with conventional sterilisation methods. This would be particularly problematic for embodiments comprising a coherent light source, which may be sensitive to, e.g., heat and disinfectants.

However, because the coupling device is connectable to the endoscope via the first light guide, the light needs to be coupled into the first light guide with sufficient intensity. This is particularly relevant for the incoherent light. Therefore, the coupling device may be configured to minimise light losses, in particular incoherent light losses, between the optical input and the optical output.

The coherent light is generated in the coupling device itself.

On the other hand, using a first coherent light source inside the coupling device may result in higher quality coherent light (e.g., higher power, smaller spectral bandwidth and/or a longer coherence length), as the coherent light may propagate through free space to the optical output or at least to the optical coupler, without having to use optical components such as optical fibres which may be detrimental to the quality of the coherent light. As is described below in more detail, the optical coupler may additionally comprise a second coherent light source and/or a coherent light input for receiving coherent light of a second wavelength.

The optical coupler may be a static optical coupler for combining a relatively wide incoherent light bundle and a relatively narrow coherent light bundle on a single optical path, and injecting both the incoherent light and the coherent light into a first light guide of a light delivery system of an endoscopic system via an optical output. Alternatively, the optical coupler may be an optical switch for (selectively) injecting either the coherent light bundle or the incoherent light bundle or both into the first light guide.

The optical coupler is an optical switch configured for switching between a first state wherein the coherent light is not injected into the first light guide, and a second state wherein the coherent light is injected into the first light guide. This way, an operator may select whether or not to use the laser light, without having to remove the coupling device.

In an embodiment, in the first state, the optical switch may be configured to provide the incoherent light at the optical output. In the second state, the optical switch may be configured to not provide the incoherent light at the optical output. In many applications, a user may want to switch between only incoherent light and only coherent light.

In an embodiment, the optical switch may be configured for switching to a third state, in which third state the optical switch may be configured to provide both the coherent light and the incoherent light at the optical output. Imaging a target area with both coherent and incoherent light simultaneously may be particularly advantageous when the coherent light is outside the visible spectrum, e.g., infrared or ultraviolet light.

In an embodiment, the optical switch may comprise a first movable, e.g., rotatable, mirror, the mirror being movable between a first position in which a reflective surface of the first mirror is positioned to reflect incoherent light propagating over the incoherent light beam path, and a second position, in which the reflective surface of the first mirror is positioned to reflect the incoherent light in a direction away from the incoherent light beam path. Additionally or alternatively, the optical switch may comprise a second mirror movable between a first position in which a reflective surface of the second mirror is positioned to reflect coherent light propagating over the coherent light beam path, and a second position, in which the reflective surface of the first mirror is positioned to reflect the coherent light in a direction away from the coherent light beam path.

The second mirror may be the same mirror as the first mirror, a different part of the same mirror, or a separate mirror. Moving mirrors may be cheap and easy to manufacture.

In an embodiment, the optical switch may comprise a first switchable mirror, switchable between a transparent state and a reflective state, positioned to reflect, in the reflective state, incoherent light propagating over the incoherent light beam path. Additionally or alternatively, the optical switch may comprise a second switchable mirror, switchable between a transparent state and a reflective state, positioned to reflect, in the reflective state, coherent light propagating over the coherent light beam path.

The first and/or second switchable mirror may be, e.g., an electrically switchable mirror. Switching a mirror between a transparent state and a reflective state may be faster than moving the mirror, and the absence of moving parts may reduce pollution of optical elements in the optical switch. The first and second switchable mirrors may be the same mirror or may be different mirrors.

In an embodiment, the optical switch may comprise a first light beam blocker configured to block the coherent light in the first state, preferably the first beam blocker comprising a mechanical shutter, preferably a rotating shutter, more preferably a motorised rotating shutter, or an optical beam blocker, preferably one of: a liquid crystal light valve, an acousto-optical modulator, an electro-optical modulator or a photo-elastic modulator.

Optionally, the optical switch may further comprise a second light beam blocker configured to block the incoherent light in the second state, preferably the second beam blocker comprising a mechanical shutter, preferably a rotating shutter, more preferably a motorised rotating shutter, or an optical beam blocker, preferably a liquid crystal light valve.

Mechanical shutters are easy and cheap to manufacture and operate. In some embodiments, both the coherent light and the incoherent light may be switched using shutters. By using rotating shutters in counter phase, alternating illumination with incoherent light and coherent light may be obtained at a relatively high frame rate. This may allow for e.g., multiplexing coherent light images and incoherent light images.

Optical beam blockers do not comprise moving parts and are thus dust-free. They are also typically fast and relatively simple to operate electronically.

In an embodiment, the optical coupler may comprise a first convergent lens to reduce divergence of the incoherent light from the optical input and a second convergent lens to focus the incoherent light into the first light guide (or, on the optical output); and a mirror, positioned between the first lens and the second lens, configured to steer the laser beam into the first light guide (or, on the optical output).

Using lenses to focus the incoherent light into the first light guide results in very little light loss. In an embodiment, one or more lenses may be replaced by mirrors, e.g., a converging lens may be replaced by a concave mirror, optionally comprising a hole or window to allow for an unobstructed coherent light beam path through the concave mirror.

In an embodiment, the optical coupler may comprise a tapered light pipe having a wide end for receiving the incoherent light from the optical input and a narrow end for providing the incoherent light to the optical output. The tapered light pipe may be divided into a first part and a second part by a plane intersecting the tapered light pipe between the wide end and the narrow end, a normal of the plane defining a non-zero angle with a longitudinal axis of the tapered light pipe. Preferably, the tapered light pipe may further comprise a protrusion having a surface normal to the coherent light path for receiving the coherent light. The optical coupler may further comprise a mirror, positioned on the plane between the first part and the second part of the tapered light pipe on the longitudinal axis of the tapered light pipe, configured to steer the laser beam into the first light guide (or, onto the optical output).

Using a tapered light pipe results in little chromatic aberrations. The protrusion may increase the amount of coherent light entering the light guide.

Both the embodiment with a tapered light pipe and the embodiment with lenses allow switching the coherent light and incoherent light independently using mechanical or optical shutters in the respective beam paths. Thus, incoherent light and coherent light may be used either separately or combined.

In an embodiment, the optical switch may comprise an internal light guide for guiding the incoherent light from a first end of the internal light guide to a second end of the internal light guide. The optical switch may further comprise a switching body comprising at least the second end of the internal light guide, the switching body being movable between a first position corresponding to the first state and a second position corresponding to the second state. In the first position, the switching body may be configured to block the coherent light beam path, to position the first end of internal the light guide for receiving incoherent light from the optical input, and to position the second end for injecting the incoherent light into the first light guide. In the second position, the switching body may be configured not to block the coherent light beam path, and to position at least the second end of the internallight guide such that the incoherent light is not injected into the first light guide.

For example, the beam path from the first coherent light source to the optical output may be a path through free space. In the first position, the light guide may be configured to guide the incoherent light to optical output, simultaneously blocking the beam path of the coherent light. By moving the light guide to a different position, the coherent light beam path to the optical output may be unobstructed. This way, very few optical components are used to provide the coherent light at the optical output, and hence, into the first light guide, which is beneficial for the spectrum and coherence length of the coherent light.

The use of an internal light guide for transporting the incoherent light minimizes light loss and chromatic aberrations. Although some incoherent light sources may automatically compensate a reduced incoherent light intensity, this compensation is typically limited. Thus, even is such systems, it is preferably to minimize light loss.

In an embodiment, the internal light guide may be a flexible light guide, preferably a fused fibre bundle or a liquid light guide, and the first end of the internal light guide may be connected to the optical input. By connecting the light guide to the optical input, the number of interfaces between light guide segments may be reduced, which may be beneficial for the light intensity and light quality of the incoherent light.

In an embodiment, the optical switch may be an electrically operated switch with a motor, optionally a servomotor; and the optical switch may be moved between a first position corresponding to the first state and a second position corresponding to the second state. The switching body may be moved via a cam or a crank. In a different embodiment, the optical switch may be operated manually.

A manually operated switch may be simpler and cheaper to manufacture than an electrically operated one. An electrically operated switch may be substantially faster, and easier to remotely operate, than a manually operated one. An electrically operated switch may comprise a motor to move a movable part of the optical switch, e.g., a movable mirror or the switching body, between the first position and the second position. A servomotor may be used to quickly and accurately move the optical switch.

In an embodiment, the optical switch may comprise a first end stop configured to stop the switch in a first position corresponding to the first state; and a second end stop configured to stop the switch in a second position corresponding to the second state. The optical switch may further comprise a spring configured to keep the switch in the first or second position if the switch is not being operated. This configuration is reliable and accurate, and may be cheaper than using a servomotor.

In an embodiment, the optical switch is configured to switch automatically back and forth between the first state and the second state, preferably with a frequency of at least 10 Hz, more preferably with a frequency of at least 30 Hz, even more preferably with a frequency of at least 100 Hz.

By alternating between two illumination states, alternating images may be acquired. This may be beneficial for further processing, where e.g., images based on coherent light imaging may be overlaid on the preceding or subsequent incoherent light image, thus combining information from two imaging modalities.

In an embodiment, the optical input connector is configured for connecting, preferably releasably connecting, to an incoherent light output of the endoscopic system via a light pipe; that is, the second light guide may be a light pipe. In a different embodiment, the optical input connector is configured for connecting, preferably releasably connecting, to an incoherent light output of the endoscopic system via a flexible light guide, preferably a fibre bundle, a fused fibre bundle, or a liquid light guide; that is, the second light guide can be a flexible light guide. A rigid connection using a light pipe results in very little light loss, while a flexible connection using an optical fibre allows for a more flexible placement of the coupling device relative to the incoherent light source of the endoscopic system.

In an embodiment, the first light source is a narrow-bandwidth laser, preferably having a spectrum bandwidth of less than 1 nm, more preferably less than 0.2 nm, even more preferably less than 0.1 nm.

In an embodiment, the first light source may have a coherence length of at least 0.35 mm, preferably at least 1.5 mm, more preferably at least 3.5 mm.

In an embodiment, the first light source may be a laser with a power output of at least 20 mW, preferably at least 100 mW, even more preferably more at least 150 mW.

High-quality laser speckle contrast images may be obtained using a coherent light source with a small spectral bandwidth and long coherence length. A coherent light source with a power output of at least 20 mW may prevent underexposure during imaging, depending on the site to be imaged.

In an embodiment, the first wavelength may be selected in the red part of the electromagnetic spectrum, preferably in the range 600-700 nm, more preferably in the range 630-660 nm. In another embodiment, the first wavelength may be selected in the infrared part of the electromagnetic spectrum, preferably in the range 700-1200 nm, more preferably in the range 700-900 nm, even more preferably in the range 770-790 nm or in the range 820-840 nm.

For laser speckle contrast images, e.g., perfusion images, red light is preferred, as they have a relatively large penetration depth and are well reflected by red blood cells. If an RGB camera or equivalent component of the endoscopic system is used, the first wavelength may be selected to be imaged by the red channel of the RGB camera. Infrared light may also be used for laser speckle contrast imaging, and has a larger penetration depth than red light. Infrared light may also be used for ICG-based fluorescence imaging. Infrared light imaging may be combined with simultaneous white light imaging. A further advantage of using infrared light is that laser speckle contrast imaging and ICG-based fluorescence imaging may be performed simultaneously.

Some endoscopes may comprise filters blocking infrared light and/or may not be configured for detecting infrared light; thus, using coherent light in the visible spectrum may allow for using the coupling device or imaging system with a wider range of endoscopes.

In an embodiment, the coupling device may further comprise a second light source for generating light of a second wavelength; and an optical combiner for combining the light of the first wavelength with the light of the second wavelength, the optical combiner preferably comprising a dichroic mirror for selectively reflecting the light of the first wavelength or the light of the second wavelength.

A second coherent light source may be advantageous for laser speckle contrast images, as images obtained using the second wavelength may be used to correct images obtained using the first wavelength.

In an embodiment, the second wavelength may be selected in the blue or green part of the electromagnetic spectrum, preferably in the range 380-590 nm, more preferably in the range 470-570 nm, even more preferably in the range 520-560 nm.

For laser speckle imaging, it is advantageous if the second wavelength has a small penetration depth and is to a large extent reflected by tissues that are being imaged. If an RGB camera or equivalent component of the endoscopic system is used, the second wavelength may be selected to be imaged by the green or blue channel of the RGB camera. Preferably, the first and second wavelength are far enough apart on the electromagnetic spectrum to limit crosstalk between the RGB channels.

The endoscopic system may further comprise a camera, preferably an RGB camera, the camera being configured to provide a video signal. In an embodiment, the coupling device may further comprise: a video signal input connector for receiving the video signal; a video signal output connector for providing a video output; and an image processing module for generating coherent light images, preferably fluorescence images and/or laser speckle contrast images based on the video signal when the optical coupler injects coherent light into the first light guide.

In general, instead of an RGB camera, dual or triple monochrome cameras, e.g., CCD cameras, may be used with, for example, different colour filters, e.g., red and green filters, or a wavelength-selective beam splitter, e.g., a prism. Alternatively, a single monochrome camera with a mechanically, optically, or electrically switchable colour filter or other wavelength-based selection device may be used. In such cases, a red channel of an RGB camera may be read as, e.g., a monochrome camera configured to measure light in the red part of the electromagnetic spectrum and, mutatis mutandis, the same for references to the green and blue channels, as the case may be. This relates both to cameras in endoscopic systems and to cameras in imaging systems according to an embodiment of the invention.

In an embodiment, in the first state, the video signal may be looped to the video output. In the second state, the video signal may be provided to the image processing module. The image processing module may be configured to determine one or more output images, preferably laser speckle contrast images or fluorescence images, based on the video signal. The one or more output images may be provided at the video signal output connector.

Including an image processing module prevents the need to use a separate image processing module to process the coherent light images.

In an embodiment, the coupling device may further comprise a frame grabber for converting a continuous video input signal into discrete frames for processing by the video processing module.

In an embodiment, the coupling device may further comprise a first sensor, preferably an optical sensor, and a controller, connected to the first sensor. The first sensor may be configured to send a first signal to the controller only if the first light guide is connected to the optical output connector; and the controller may be configured to switch off or block the first coherent light source and, optionally, the second coherent light source, if the controller does not receive the first signal.

In an embodiment, the coupling device may further comprise a second sensor, preferably an optical sensor, and a controller, connected to the second sensor. The second sensor may be configured to send a second signal if the optical switch is in the second state; and the controller may be configured to switch off or block the first coherent light source and, optionally, the second coherent light source, if the controller does not receive the second signal.

Such sensors may increase the safety of the coupling device, by preventing coherent light from exiting the coupling device if there is the first light guide is not connected to deliver the coherent light.

In an embodiment, the coupling device may further comprise a control switch for generating a trigger signal or a trigger input for receiving a trigger signal, wherein the optical switch is configured to switch from the first state to the second state in response to receiving a trigger signal.

In an embodiment, the coupling device may further comprise a data storage medium or a data output.

The endoscopic systems described in this disclosure are not limited to medical endoscopic systems, but may also include similar systems comprising an incoherent light source and a light delivery system from other fields, e.g., manufacturing, gunsmithing, or aviation. Endoscopic systems in non-medical fields are sometimes referred to as borescopes or fiberscopes. They may be used for visual inspection of e.g., engines or the interior bore of a firearm.

In a further aspect, the invention may relate to an image processing system for generating coherent light images with an endoscopic system. The endoscopic system may comprise an incoherent light source, a video processing unit, and an endoscope, with the endoscope being connected to a camera. In an embodiment, the image processing system may comprise a coupling device according to one of the embodiments described above, and a video processing device. The video processing device may comprise a video signal input connector for receiving a video signal from the camera. The video processing device may further comprise a first video signal output connector for providing a video output, the first video signal output connector configured to be connected to a video signal input of the video processing unit of the endoscopic system. The video processing device may also comprise an image processing module for generating coherent light images based on the video signal when the optical switch is in the first state.

In an embodiment, the image processing system may further comprise a second video signal output connector configured to be connected to a second display, the image processing system being configured to provide an incoherent light image to the first video output when the optical switch is in the first state and to provide a coherent light image to the second video signal output when the optical switch is in the second state.

In an aspect, the invention may relate to an imaging system for generating an infrared coherent light image with an endoscopic system, the endoscopic system comprising an incoherent light source, an endoscope, and a light delivery system for delivering light to the endoscope. In an embodiment, the imaging system may comprise a coupling device as described above, the first wavelength being selected in the infrared part of the electromagnetic system, preferably in the range 700-1200 nm, more preferably in the range 700-900 nm, even more preferably in the range 770-790 nm or in the range 820-840 nm. The imaging system may further comprise an infrared imaging sensor configured to receive light collected by the endoscope, and an image processing module configured to receive a video signal from the infrared imaging sensor and to determine a coherent light image based on the received video signal.

The infrared imaging sensor may be included in an RGB/IR camera or equivalent device, i.e., a camera configured to capture images in both the visible light and the infrared part of the electromagnetic spectrum using a single sensor array. Alternatively, the imaging sensor may further comprise a beam splitter for splitting the light reflected by the target into an infrared part imaged by the infrared imaging sensor, and a visible light part imaged by an RGB camera included in either the endoscopic system or the imaging system, as described above.

In an aspect, the invention may relate to an endoscopic system, preferably a laparoscopic system, comprising the coupling device as described above.

In an aspect, the invention may relate to a method for generating a coherent-light-based image of a target area in a patient body using an endoscopic system. The endoscopic system may comprise an incoherent light source for generating incoherent light, e.g., white light, and an endoscope, the endoscope comprising an insertion tube for inserting in the patient body, a light delivery system for illuminating the target area, and an image sensor for acquiring an image of the target area. The endoscope may be connected, preferably releasably connected, to a coherent light coupling system via a first light guide, the coherent light coupling system being configured to receive the incoherent light from the incoherent light source via a second light guide, receive or generate coherent light of a first wavelength, and selectively provide the coherent light and/or the incoherent light to the first light guide, wherein the coherent light and the incoherent light preferably have a shared optical path in the first light guide. In an embodiment, the method may comprise receiving a first trigger signal and, in response to receiving the trigger signal, providing coherent light to the light delivery system. The method may further comprise receiving a video stream from the image sensor, the video stream comprising a signal representing a light intensity of the coherent light reflected or dispersed by the target area. The method may further comprise determining a coherent light image, e.g., a laser speckle contrast image or fluorescence image, based on the video stream.

In an embodiment, the method may further comprise displaying and/or storing the determined coherent light image.

In an embodiment, the method may further comprise, in response to receiving a second trigger signal, or a predetermined amount of time after receiving the first trigger signal, providing the incoherent light to the light delivery system and, preferably, blocking the coherent light from entering the light delivery system.

The invention may also relate to a computer program or suite of computer programs comprising at least one software code portion or a computer program product storing at least one software code portion, the software code portion, when run on a computer system, being configured for executing one or more of the method steps described above.

The invention may also relate to a non-transitory computer-readable storage medium storing at least one software code portion, the software code portion, when executed or processed by a computer, is configured to perform one or more of the method steps as described above.

The invention will be further illustrated with reference to the attached drawings, which schematically will show embodiments according to the invention. It will be understood that the invention is not in any way restricted to these specific embodiments.

### Brief description of the drawings

The following description of the figures of specific embodiments of the invention is merely exemplary in nature and is not intended to limit the present teachings, their application or uses.
**Fig. 1** schematically depicts an endoscopic system enhanced with a coupling device to provide coherent light imaging according to an embodiment of the invention;
**Fig. 2A** and **2B** schematically depict a coupling device according to an embodiment of the invention;
**Fig. 3** schematically depicts a coupling device according to an embodiment of the invention;
**Fig. 4A** and **4B** schematically depict a coupling device according to an embodiment of the invention;
**Fig. 5A** through **5D** schematically depict optical switches according to embodiments of the invention;
**Fig. 6A** through **6C** schematically depict optical switches according to embodiments of the invention;
**Fig. 7A** and **7B** schematically depict connections between a light source unit of an endoscopic system and a coupling device according to an embodiment of the invention;
**Fig. 8** schematically depicts a system for adding coherent light imaging capability to an endoscopic system according to an embodiment of the invention; and
**Fig. 9** depicts a method for generating a coherent light image according to an embodiment of the invention.

### Detailed description

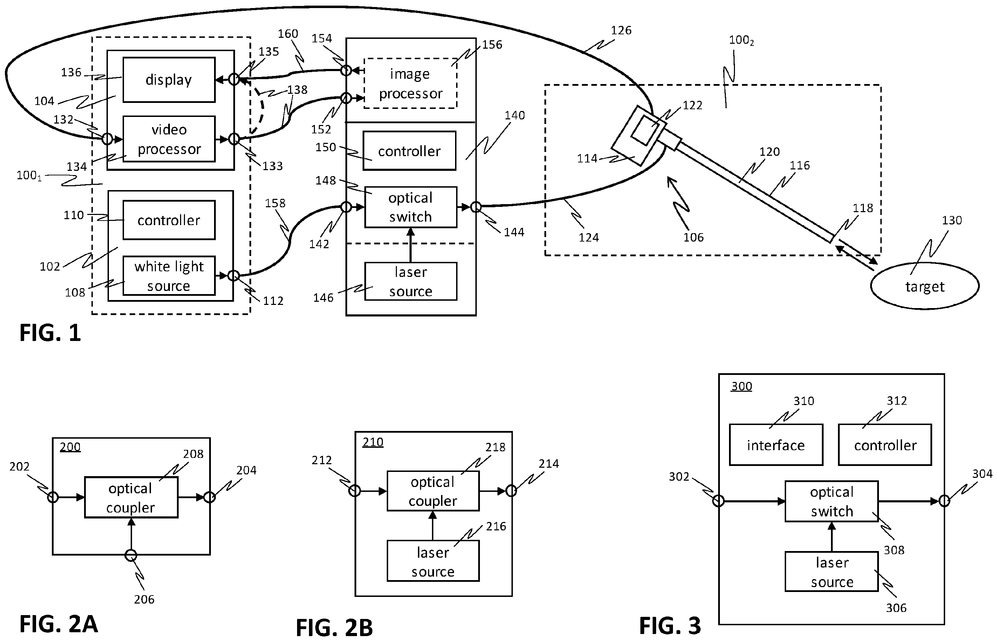

**Fig. 1** schematically depicts an endoscopic system enhanced with a device to provide coherent light imaging according to an embodiment of the invention. The endoscopic system **100₁,100₂** may comprise a light source unit **102**, a display unit **104,** and an endoscope **106,** e.g., a laparoscope.

The light source unit **102** may comprise a light source **108** for generating incoherent light, e.g., a white light source. The light source unit may further comprise a controller **110** for controlling the light source, e.g., controlling the light intensity or the spectral qualities of the light source. The controller may be configured to receive input from a user and/or from other system components. The light source unit may further comprise an optical output connector **112** for connecting an optical input, e.g., light guide **124,** of the endoscope **106.**

The endoscope **106** may comprise a handle **114** for operating the endoscope and a insertion tube **116.** The insertion tube may have a proximal end connected to the handle and a distal end with a tip **118** for being inserted into a patient. The insertion tube may be a rigid tube or shaft or a flexible tube.

The endoscope may further comprise an optical input for receiving light via an input light guide **124,** e.g., an optic fibre bundle. The light input may be connectable to the handle or to the insertion tube.

The insertion tube may comprise a light guide **120** for guiding light from the optical input to the tip **118** for illuminating a target area **130.** The optical components between the optical output **112** of the light source unit and the tip of the endoscope may collectively be referred to as a light delivery system of the endoscopic system.

The endoscope may further comprise an image sensor **116,** e.g., an RGB camera, for imaging the target area. In some endoscopes, the image sensor may be placed at the tip of the endoscope; such systems may also be referred to as chip-on-the-tip systems. In other endoscopes, the image sensor may be placed in the handle and the insertion tube may comprise an optical guide, e.g., a rod lens, for guiding light reflected or emitted by the target area to the optical sensor.

In some endoscopes, the handle **114** and the insertion tube **116** may be detachable. Thus, it may be possible to exchange the image sensor **122** for a different type of image sensor, while retaining the same insertion tube **116.**

The endoscope may be connectable to a video input connector **132** of the display unit **104** using a video signal cable **126.** The endoscope may further comprise wired or wireless connections for e.g., remotely controlling the light source unit **102** and/or the display unit **104.** For example, the video signal cable may also be a data cable allowing bidirectional data transport. The endoscope may further comprise a power source or a power connector for receiving power to operate the image sensor.

The display unit **104** may comprise a video input connector **132** for receiving video input from the endoscope **106.** The display unit may further comprise a video processor **134** for processing the video input signal, and a display **136** for displaying video or images received from the endoscope. The display may receive video input from the video processor via an (external) video cable **138,** connecting a video output connector **133** of the video processor with a display input connector **135.** In other embodiments, the display may receive input from the video processor via an internal connection.

The display unit **104** may further comprise a controller configured to receive input from a user and/or from other system components, e.g., for pausing the video or taking snapshots. The display unit may also provide output to other system components; for example, the display unit may communicate with the light source unit **102** to adjust the light intensity if the image in the video input signal appear to be overexposed or underexposed. The display unit may further comprise or be connectable to a storage device for storing e.g., image data. In other endoscopic systems, the display may be separate device from the coupling device comprising the video processor. In some endoscopic systems, the video processor and the light source may be included in a single device.

Thus, the endoscopic system **100₁,100₂** described so far may be a (commercially available) endoscopic system for imaging a target area **130** in a patient body using incoherent light, e.g., white light. Certain embodiments in this disclosure relate to a device that allows to use the same, or essentially the same, endoscopic system for imaging the target area using coherent light, e.g., laser light together with incoherent light imaging or alternating with incoherent light imaging. Thus, new capabilities, in particular imaging capabilities such as fluorescence imaging and/or laser speckle contrast imaging, may be added to an existing endoscopic system, without having to exchange the light source or, at least, the insertion tube of the endoscope.

Therefore, a coupling device **140** for coupling coherent light into the light delivery system of the endoscopic system may be inserted between the optical output **112** of the light source unit and the optical input of the endoscope. The coupling device comprises an optical input **142** for receiving the incoherent light from the endoscopic system and an optical output **144** for providing the incoherent light to the light delivery system. The optical input comprises an optical input connector for releasably connecting a light guide **158.** Similarly, The optical output comprises an optical output connector for releasably connecting a light guide **124.**

In the depicted embodiment, the coupling device's optical input **142** is connectable to the optical output **112** of the endoscopic system's light source unit **102** via a light guide **158,** e.g., a fibre bundle. The coupling device's optical output **144** is connectable to an optical input of the endoscope via the light guide **124.** In a set-up without the coupling device, light guide **124** might be connected directly to the light source unit's optical output **112.** A releasable connection to connect and disconnect a light guide to a light source is a standard feature for endoscopic systems.

The coupling device **140** further comprises a first light source **146,** e.g., a laser, for generating coherent light of a first wavelength. The coupling device further comprises an optical switch **148** for selectively coupling the coherent light of the first wavelength to the optical output **144** and hence, when the light guide **124** is connected, into the light guide **124.** The coupling device may also comprise a controller for controlling the optical switch.

In an embodiment, the coupling device **140** may further comprise a video signal input connector **152** for receiving a video input signal. The video signal may be prepocessed by the video processor **134** or may be obtained directly from the image sensor **122** in the endoscope. The coupling device may also comprise a video signal output connector **154** for providing a video output signal. The coupling device may further comprise an image processing module **156** for processing the video signal. Dedicated image processing software may take advantage of the possibilities of coherent light imaging, e.g., by implementing laser speckle contrast imaging. In a different embodiment, the image processing module and the video signal input and output connectors may be embodied in a separate device.

In the depicted embodiment, the coupling device's video signal input connector **152** may be connectable to a video signal output of the display unit **104** via a signal cable **138.** In other embodiments, the coupling device's video signal input connector may be connectable directly to a video signal output of the endoscope **106.** In some such embodiments, the signal cable may allow bidirectional data transport and may for example send trigger or control signals from the endoscope to the coupling device or vice versa. The coupling device's video signal output connector **154** may be connectable to the display input connector **135** of the display unit **104** via the signal cable **160.** In a set-up without the coupling device, signal cable **138** might be connected directly to the display input connector **135.**

**Fig. 2A** and **2B** schematically depict a coupling device. In particular, **fig. 2A** depicts a very basic coupling device **200** for coupling coherent light into a light delivery system of an endoscopic system. The depicted coupling device comprises an optical input **202** for receiving incoherent light, e.g., white light, from a light source of the endoscopic system and an optical output connector **204** for connecting to a light delivery system of the endoscopic system. The coupling device further comprises a coherent light input **206** for receiving coherent light of a first wavelength. The coherent light input optionally comprises a collimator to prevent or reduce divergence of the coherent light bundle. The coupling device further comprises an optical coupler **208** for receiving the incoherent light and the coherent light and for providing a coupled light beam. The coupled light beam may comprise the coherent light and the incoherent light alternately. The coupling device is further configured for injecting the coupled light beam into the optical output **204.**

Thus, the coupling device may be configurable in a first state and a second state. In the first state, the coupling device comprises an unobstructed incoherent light beam path for propagating the incoherent light from the optical input to the optical output. In the second state, the coupling device comprises an unobstructed coherent light beam path for propagating the coherent light from the coherent light source or the coherent light input to the optical output. The optical coupler couples or combines the incoherent light beam path and the coherent light beam path onto a shared optical path configured to alternately inject the incoherent light and the coherent light into the optical output and, hence, if present, into a light guide connected to the optical output connector.

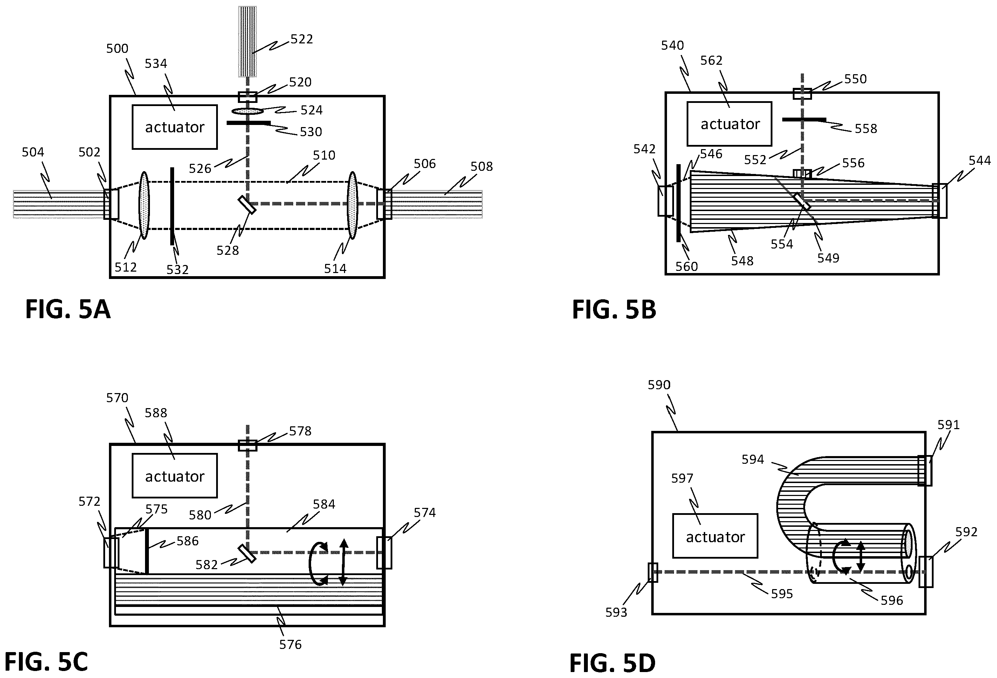

The optical coupler **208** is an optical switch for selectively coupling the coherent light into the optical output **204.** Thus, the optical output provides either the incoherent light or the coherent light. Embodiments of the optical coupler are discussed in more detail with reference to **fig. 5A-D.**

**Fig. 2B** depicts a similar coupling device **210** comprising an optical input **212**, an optical output **214**, and an optical coupler **218** as described above with reference to **fig. 2A.** The coupling device **210** further comprises a first coherent light source **216** for generating coherent light of a first wavelength.

On the other hand, when the coupling device comprises an (internal) first coherent light source as in **fig. 2B**, the coherent light may propagate through free space to the optical output, or at least to the optical coupler. This may result in higher quality coherent light (e.g., higher power, smaller spectral bandwidth and/or a longer coherence length), as there is no need to use optical components such as optical fibres which may be detrimental to the quality of the coherent light. It is typically not possible to have an external coherent light source and free space coherent light propagation because of safety issues and alignment difficulties.

**Fig. 3** schematically depicts a coupling device according to an embodiment of the invention. In particular, **fig. 3** depicts a coupling device **300** comprising an optical input **302**, an optical output **304**, and a first coherent light source **306** for generating coherent light of a first wavelength as discussed above with reference to **fig. 2A** and **2B.** The coupling device also comprises an optical switch **308** for selectively coupling the coherent light into the optical output **304**.

The coupling device **300** further comprises an interface **310** for operating the optical switch **308**. The interface may comprise a physical trigger on the coupling device, e.g., a button, or a software-based trigger, e.g., a function (virtual button) on a touch screen. In other embodiments, the interface may comprise an input for receiving a wired or wireless trigger signal from an external device, for example from a part of the endoscopic system or from an external control device. A wireless trigger signal may use e.g., WiFi, Bluetooth, or an acoustical or optical communication protocol. Some endoscopic systems may be configured to provide a trigger signal when certain functionality of the endoscopic system is activated, e.g., a photo taking function. Such a trigger signal may be used to activate the optical switch. An external control device may be operable by an operator of the endoscopic system, e.g., a surgeon or endoscopist, and may comprise a foot switch or a remote control, preferably attachable to a handle of an endoscope, e.g., using an elastic band. The interface may also comprise e.g., a microphone and voice recognition software for voice-based operation of the optical switch. The interface may further provide feedback, preferably visual feedback, regarding the state of the optical switch.

The optical switch **308** is configured for switching between a first state wherein the coherent light is not coupled the optical output **304,** and a second state wherein the coherent light is coupled to the optical output. This way, an operator may select whether or not to use the laser light, without having to remove the coupling device.

In the first state, the optical switch **308** is configured to provide the incoherent light at the optical output **304.** In the second state, the optical switch is configured to not provide the incoherent light at the optical output. In many applications, a user may want to switch between only incoherent light and only coherent light.

In some embodiments, the optical switch **308** may also be configured for switching to a third state, in which third state the optical switch may be configured to provide both the coherent light and the incoherent light at the optical output **304.**

The interface **310** may comprise a mechanical or electrical trigger. In case of a mechanical trigger, the switch may be manually operated by shifting between a first position, corresponding to the first state, and a second position, corresponding to the second state. In such an embodiment, the coupling device may not comprise a controller.

In the shown embodiment, the coupling device **300** further comprises a controller **312** for controlling the optical switch. In such an embodiment, the interface may send a trigger signal to the controller, and the controller operates the optical switch. In a typical embodiment, the optical switch is an electrically operated switch with a moving mechanical part actuated with a motor which is controlled by the controller.

The controller controls the optical switch to switch between the first state and the second state independent of a trigger signal, e.g., at fixed time intervals. In such an embodiment, the coupling device need not comprise an interface.

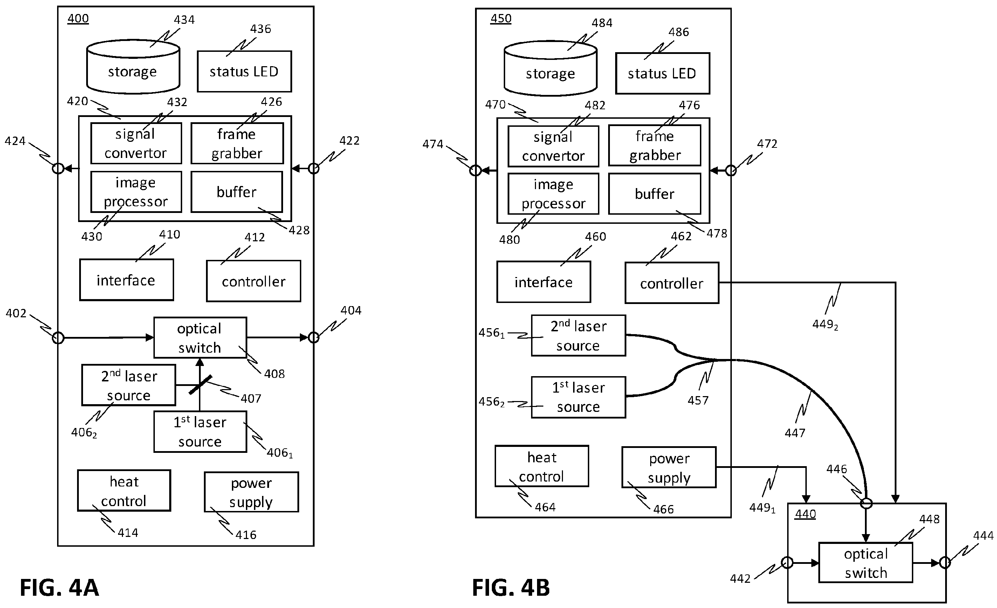

**Fig. 4A** and **4B** schematically depict a coupling device according to an embodiment of the invention. In particular, **fig. 4A** depicts a coupling device **400** comprising an optical input **402,** an optical output **404,** an optical switch **408,** an interface **410,** and a controller **412** as discussed above with reference to **fig. 2A, 2B,** and **3****.**

The coupling device further comprises a first coherent light source **406₁** for generating coherent light of a first wavelength, a second coherent light source **406₂** for generating coherent light of a second wavelength, and a laser coupler **407,** e.g., a dichroic mirror or a polarising beam splitter, for coupling the coherent light of the first wavelength with the coherent light of the second wavelength. Alternatively, fibre-based laser coupling as described hereunder with reference to **fig. 4B** may be used. Typically, the second wavelength is different from the first wavelength. By using a laser coupler, the coherent light of the first wavelength and the coherent light of the second wavelength may be combined on a single optical path.

A second coherent light source may be advantageous for laser speckle contrast images, as images obtained using the second wavelength may be used to correct images obtained using the first wavelength. A dichroic mirror is an efficient way to couple or combine the light of the second wavelength with the light of the first wavelength. In some embodiments, even more coherent light sources may be added in a similar way. This may be useful for e.g., three-dimensional laser speckle contrast imaging, based on laser speckle contrast images obtained with coherent light of a plurality of different wavelengths.

The coupling device **400** may further comprise an image processing module **420.** The image processing module may receive a video signal from a video input connector **422** and provide a video signal to a video output connector **424.** Typically, an endoscope may be connected to the video signal input connector, either directly or indirectly via, e.g., a video processor of the endoscopic system, and a display unit may be connected to the video signal output connector.

The video signal may be an analogue signal or a digital signal. The video processing module **420** may comprise a frame grabber **426** for converting a video signal into separate image suitable for further processing. The video processing module may comprise a buffer **428** for temporarily storing one or more images. The one or more images may then be processed by an image processor **430.** The image processor may comprise a computer-readable storage medium having computer-readable program code embodied therewith, and a processor, preferably a microprocessor, coupled to the computer-readable storage medium.

The image processor may be configured to compute e.g., laser speckle contrast images or fluorescence images. For example, the image processor may be configured to execute a LSCI-based clinical perfusion imaging scheme as described in the review article by W. Heeman et al, *'Clinical applications* of *laser speckle contrast imaging: a review', J. Biomed. Opt.* 24:8 (2019), which is hereby incorporated by reference. Additionally or alternatively, the image processor may be configured to execute a fluorescence-based clinical perfusion imaging scheme, preferably ICG-based fluorescence imaging, as described in the review article by A.V. DSouza et al, *'Review of fluorescence guided surgery systems: identification of key performance capabilities beyond indocyanine green imaging', J. Biomed. Opt.* 21:8 (2016), which is hereby incorporated by reference.

The video processing module **420** may further comprise a signal convertor **426** for converting the output generated by the image processor into an output video signal compatible with the input video signal. For instance, if the video processing module receives an SDI signal from the video input and the image processor outputs a HDMI signal, the image convertor may be an HDMI to SDI convertor. Thus, the video output signal is compatible with the video input signal, ensuring that it may be processed by the display unit in the same way the signal from the endoscope would be.

In an embodiment, the coupling device may comprise a screen or be connectable to a different display. In that case, a signal convertor may not be needed. Likewise, if the image processor provides output of the same type as is received from the endoscope, a signal convertor may not be needed. In an embodiment, the coupling device may have more than one video output connector, e.g., a first output connector for outputting an unprocessed video signal, and a second output connector for outputting a processed signal.

In an embodiment, the video processing module **420** may communicate with the controller **412.** The video processor module may then e.g., provide an unprocessed signal when the optical switch is in the first state, providing only incoherent light, e.g., white light, to the endoscope, and provide a processed signal when the optical switch is in the second state, providing coherent light to the endoscope. In an embodiment, the video processing module and the controller may be implemented as software modules on the same hardware module.

The coupling device **400** may further comprise a storage **434** for storing images, or a storage output connector to connect to an external storage device. The coupling device may, for instance, be configured to store all image data, to store only processed and/or unprocessed image data when the optical switch is in the second state, of only when a storage signal is received from the controller **412.**

The coupling device **400** may also comprise a power supply **416** for providing power to electrical components, e.g., the one or more laser sources **406₁₋₂**, the optical switch **408,** the controller **412,** the video processing module **420,** and any other components requiring power. One or more heat control units **414,** which may comprise e.g., heat sinks and/or fans, may control a temperature of one or more components such as the laser sources and the video processing module. One or more status LEDs **436** may indicate the status of various components, e.g., power being supplied by the power supply, the state of the optical switch, the optical output connector being engaged, et cetera. A housing may protect the components.

**Fig. 4B** schematically depicts a system comprising a coupling device **440** and a processing device **450.** The coupling device **440** may comprise an optical input **442,** an optical output **444,** and an optical switch **448** as described with reference to **fig. 4A****.** The processing device **450** may comprise an interface **460,** a controller **462,** a heat control **464,** a power supply **466,** a video input connector **472,** a video output connector **474,** storage means **484,** one or more status LEDs **486,** and a video processing module **470** comprising a frame grabber, a buffer, an image processor and a signal convertor, also as described with reference to **fig. 4A****.**

The processing device **450** may further comprise a first coherent light source **456₁**, a second coherent light source **456₂**, and a laser coupler **457.** In the depicted example, the laser coupler comprises a fibre coupler. As the coherent light is guided from the processing device **450** to the coupling device **440** using an optical fibre **447,** this is an efficient way of combining the plurality of laser sources. In other embodiments, the first coherent light and the second coherent light might first be combined using a laser coupler as explained with reference to feature **407** in **fig. 4A****,** and the coupled or combined coherent light bundle might subsequently be injected into a fibre.

The optical fibre **447** may be connectable to a coherent light input **446** of the coupling device **440.** Optionally, the coherent light input may comprise a collimator. The coupling device may further be connectable to the processing device **450** by one or more cables **449₁₋₂** for receiving power and control signals to operate the optical switch **448.**

**Fig. 5A** through **5D** schematically depict optical switches according to embodiments of the invention. Some of the embodiments described below with reference to **fig. 5A** through **5D** may be implemented as non-switching optical couplers. These embodiments are typically more light-efficient than e.g., a Y-cable coupler as described above with reference to **fig. 2A.**

In particular, **fig. 5A** depicts an optical switch **500** comprising an incoherent light input **502,** a coherent light input **520,** and a light output **506.** The incoherent light input may be configured to receive incoherent light via a first light guide **504,** e.g., a light pipe or a fibre bundle. The incoherent light input may be an optical input of a coupling device or may be optically connected to the optical input. The light output may be an optical output of the coupling device or may be optically connected to the optical output. Thus, the light output may provide light to a second light guide **508,** which may be an internal light guide or a light guide of an endoscopic system.

The optical switch **500** may further comprise a first convergent lens **512** to reduce divergence of the incoherent light **510** from the coherent light input **502** and a second convergent lens **514** to focus the incoherent light on the optical output **506.** In an alternative embodiment, one or more convergent lenses may be replaced by one or more concave mirrors. Concave mirrors may have superior optical qualities than lenses, e.g., less light absorption and/or less chromatic aberration; however, lenses may be cheaper than concave mirrors. It is evident to the skilled person that when concave mirrors are used, the position of the incoherent light input and, optionally, of the coherent light input, may be adjusted accordingly.

The optical switch **500** may further be configured to receive coherent light from a coherent light source **522** or via an third light guide, e.g., an optical fibre. If the coherent light propagates through free space, the coherent light input **520** may be simply a hole or a window in a housing of the optical switch. In some embodiments, in particular when the coherent light input receives coherent light via a third light guide, the optical switch may comprise a third convergent lens **524** to reduce divergence of coherent light bundle **526.**

The optical switch **500** may further comprise a mirror **528,** positioned between the first convergent lens **512** and the second convergent lens **514.** The mirror may be configured to steer the coherent light beam **526** onto the light output **506.** As the coherent light bundle is much smaller in diameter than the incoherent light bundle **510,** the mirror may be small relative to the diameter of the incoherent light bundle. The mirror may be mounted using a small, optionally transparent, holder. Thus the intensity loss of the incoherent light may be small. As both the incoherent light **510** and the coherent light **526** travel through free space between the light inputs **502,520** and the light output **506,** intensity loss is further minimised.

The optical switch **500** may further comprise a first shutter **530,** preferably a rotational shutter, positioned between the coherent light input **520** and the mirror **528.** The first shutter may be configured to move between a first state blocking the coherent light bundle **526** and a second state not blocking the coherent light bundle. By closing and opening the first shutter, the optical switch switches between a first state in which the coherent light is not coupled to the light output, and a second state in which the coherent light is coupled to the light output.

In an alternative embodiment, the first shutter **530** may be a non-mechanical light blocking mechanism e.g., a liquid crystal light valve, an acousto-optical modulator, an electro-optical modulator such as a Pockels cell, or a photo-elastical modulator. In some cases, the coherent light may need to be polarised, e.g., by using a polarised laser source or by inserting a polariser between the coherent light source and the first shutter.

In another embodiment, the coherent light beam **526** may be steered onto or away from the optical output **506** by moving mirror **528.** In such an embodiment, the first shutter **530** may be removed. Alternatively, the mirror may be a switchable mirror, e.g., an electro-optically switchable mirror. The switchable mirror may be switched between a transparent state in the first state of the optical switch, and a reflective state in the second state of the optical switch. Depending on the size of the mirror, the movable or switchable mirror may also be used to block the incoherent light in the second state, thus removing the need for the second shutter **532.**

By selecting a suitable placement for the incoherent light input and the coherent light input, a single mirror may be used to reflect either the incoherent light or the coherent light onto the optical output.

In yet another embodiment, the coherent light may be switched on and off by switching on and off a coherent light source. This way, there is no need to include a moving part. However, using a shutter may be faster than switching the coherent light source.

Optionally, the optical switch **500** may comprise a second shutter **532,** preferably a rotational shutter, positioned between the incoherent light input **502** and the mirror **528.** The second shutter may be configured to move between a first state not blocking the incoherent light bundle **510** and a second state blocking the incoherent light bundle. Alternatively, the second shutter may be implemented as a liquid crystal light valve.

The first shutter **530** and, optionally, the second shutter **532** may be moved by one or more actuators **534.**

In an embodiment, the first shutter **530** and the second shutter **532** may be operated jointly, such that when the first shutter blocks the coherent light bundle **526,** the second shutter does not block the incoherent light bundle **510,** and vice versa.

The first shutter **530** and the second shutter **532** may be rotating shutters, allowing fast switching between the first state and the second state. By using rotating shutters in counter phase, alternating illumination with incoherent light and coherent light may be obtained at a relatively high frame rate.

Thus, the optical switch **500** may be configured to switch automatically back and forth between the first state and the second state, preferably with a frequency of at least 10 Hz, more preferably with a frequency of at least 30 Hz, even more preferably with a frequency of at least 100 Hz. By alternating between two illumination states, alternating images may be acquired. This may be beneficial for further processing, where e.g., images based on coherent light imaging may be overlaid on or shown side to side with the preceding or subsequent incoherent light image, thus combining information from two imaging modalities.

In a different embodiment, a static optical coupler could be obtained by removing the shutters **530,532** and the actuator **534.**

In another embodiment, the positions of the incoherent light input **502** and the coherent light input **520** may be exchanged, and mirror **528** may be configured to reflect incoherent light from the incoherent light input to the light output **506.** The mirror may further comprise a hole or window to allow the coherent light bundle to propagate from the coherent light input to the light output.

In yet another embodiment, both the incoherent light input **502** and the coherent light input **520** may be placed under an angle with respect to the light output **506,** and the mirror **528** may comprise two or more reflecting surfaces for reflecting coherent light and/or incoherent light onto the light output.

In yet another embodiment, the coherent light input may be placed close to the incoherent light input, e.g., both on a wall opposite the light output, and the coherent light and/or the incoherent light may be arranged to inject light under a small angle into the light output. In such an embodiment, the mirror may be removed. However, embodiments including a mirror may be easier to align and may have a less light loss, as both the coherent light and the incoherent light may be injected into the optical output under an optimal angle.

**Fig. 5B** depicts an optical switch **540** comprising an incoherent light input **542,** a coherent light input **550,** and a light output **544** as described with reference to **fig. 5A****.** The optical switch **540** may further comprise a first shutter **558,** a second shutter **560,** and an actuator **562** as described with reference to **fig. 5A****.**

However, instead of lenses **512** and **514** as depicted in **fig. 5A****,** the optical switch **540** may comprise a tapered light pipe **548** having a wide end for receiving incoherent light **546** from the incoherent light input **542** and a narrow end for providing the incoherent light and/or the coherent light to the light output **544.** The incoherent light bundle exiting the incoherent light input is typically strongly divergent. The optimum width of the wide end is therefore dependent on the distance between the incoherent light input and the wide end.

The tapered light pipe **548** may be divided into a first part and a second part by a plane **549** intersecting the tapered light pipe between the wide end and the narrow end, a normal of the plane defining a non-zero angle with a longitudinal axis of the tapered light pipe. The optical switch may further comprise a mirror **554,** positioned on the plane and configured to steer a coherent light beam **552** on the light output **544.** The tapered light pipe may comprise a protrusion **556** having a surface normal to the coherent light path for minimising light reflection by the surface of the tapered light pipe.

Using a tapered light pipe instead of lenses may minimise chromatic aberrations, in particular of the incoherent light.

**Fig. 5C** depicts an optical switch **570** comprising an incoherent light input **572,** a coherent light input **578,** and a light output **574** as described with reference to **fig. 5A****.**

The optical switch **570** may further comprise a switching body **584** which may be moved between a first position corresponding to a first state of the optical switch **570** and a second position corresponding to a second state of the optical switch. In the first state, the optical switch may be configured to provide only incoherent light at the light output **574,** while in the second state, the optical switch may be configured to provide only coherent light at the light output. The figure depicts the optical switch in the second state.

The switching body **584** may comprise a light guide **576,** e.g., a light pipe, configured to guide the incoherent light from the incoherent light input **572** to the light output **574** when the switching body is in the first position. In the second position, the incoherent light **575** may be blocked by a shutter **586.** Thus, in the second position, the switching body is configured not to guide the incoherent light to the optical output.

The switching body **584** may further comprise a mirror **582** configured to steer a coherent light beam **580** on the light output **574** when the switching body is in the second position. The mirror may further be configured not to steer the coherent light beam on the light output when the switching body is in the second position.

The switching body **584** may be moved between the first position and the second position by an actuator **588.**

In an embodiment, the optical switch **570** may comprise a first end stop configured to stop the switching body **584** in the first position corresponding to the first state; and a second end stop configured to stop the switching body in the second position corresponding to the second state. The optical switch may further comprise a spring configured to keep the switching body in the first or second position if the switch is not being operated. This configuration is reliable, accurate, and relatively cheap.

Alternatively, a servomotor may be used for moving the switching body **584** between the first position and the second position. In that case, end stops may not be needed. Using a servomotor may be faster and more silent than using a (normal) motor and end stops.

In yet another alternative, the switching body **584** may be moved manually, using e.g., a lever. This is a cheap, but relatively slow and potentially less accurate way of switching.

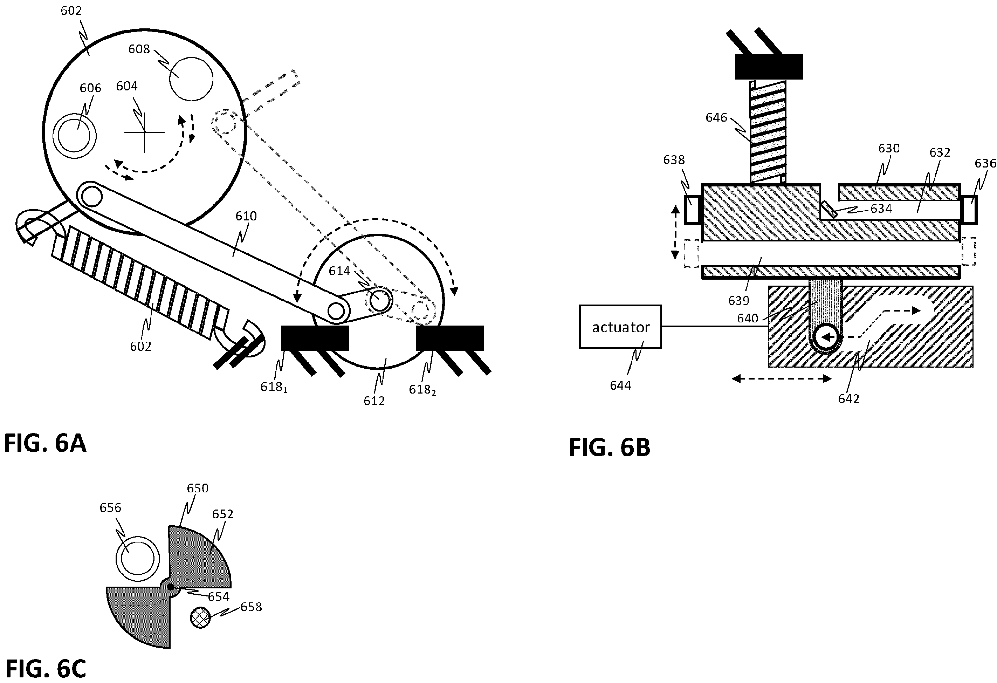

The switching body **584** may be moved between the first and second positions by e.g., a translating or rotating movement. In an embodiment, motion may be transferred to the switching body via a cam or via a crank. This is discussed in more detail below with reference to **fig. 6A** and **6B****.**

The use of light guide **576** introduces little light loss and minimises chromatic aberrations. Alternatively, a system with lenses as depicted in **fig. 5A** may be used.

**Fig. 5D** depicts an optical switch **590** comprising an incoherent light input **591,** a coherent light input **593,** and a light output **592** as described with reference to **fig. 5A****.**

The optical switch **590** may further comprise a switching body **596,** configured to be moved between a first position and a second position. The switching body may comprise at least an end of a flexible light guide **594,** e.g., a fibre bundle, a fibre bundle with fused ends, or a liquid light guide. In the first position, the switching body may be configured to guide incoherent light from the incoherent light input to the light output via the flexible light guide. The flexible light guide may extend beyond the incoherent light input and may e.g., be connectable directly to an optical output of an endoscopic system's light source unit. An advantage of this configuration is that coupling device may have an optical input and an optical output on the same side, e.g., the front side, which facilitates operation of the coupling device.

In the second position, the switching body **596** may be configured to provide coherent light **595** to the light output **592.** In the depicted example, the beam path from the coherent light input **593** to the light output **592** may be a path through free space. In the second position, the coherent light beam path to the optical output may be unobstructed. The switching body may have a hole for letting through the coherent light. Alternatively, the switching body may have a size and shape such that in the second position, no part of it covers the path between the coherent light input and the light output.

This way, very few optical components are used to provide the coherent light at the optical output, which is beneficial for the spectrum and coherence length of the coherent light.

In a different embodiment, an optical fibre may be used to guide coherent light from the coherent light input **593** to the light output **592**.This may further increase the flexibility of the setup, and may make the coupling device less sensitive to misalignment of components.

The optical switch **590** may further comprise an actuator **597,** end stops, a spring, et cetera, as described with reference to **fig. 5C****.**

**Fig. 6A** through and **6C** schematically depict optical switches according to embodiments of the invention. In particular, **fig. 6A** depicts a driving mechanism for a switching body **602** of an optical switch. The switching body may be configured to rotate around an axis **604.** As discussed in more detail above with reference to **fig. 5C** and **5D****,** the switching body may comprise a first light guide **606,** e.g., a light pipe, a fibre bundle, or a liquid light guide, configured to guide incoherent light from an incoherent light input to a light output of the optical switch when the switching body is in a first position (depicted). The switching body may further comprise a second light guide **608,** e.g., a hole or an optical fibre, configured to guide coherent light from a coherent light input to the light output of the optical switch when the switching body is in a second position (indicated with dashed lines). When the coherent light propagates through free space, the switching body may be shaped such that it does not block the coherent light beam in the second position, and does not need to comprise a hole.

The switching body **602** may be connected to a motor **612** via a crank **610.** One end of the crank may be rotatably connected to the switching body **602.** Another end of the crank may be rotatably connected to an off-axis connection point of the motor. In the first position, the crank may be pulled against a first end stop **618₁** by a spring **620,** in this embodiment attached to the switching body. In other embodiments, the spring may be attached to a different part. The spring may also push instead of pull. The end stop and spring ensure the switching body is accurately positioned in the first position. In different embodiments, the spring and, optionally, the end stop may be left out.

When the motor receives **612** a switching signal, the motor may rotate around axis **614** to a second position. In the second position, a part of the crank or another suitable component may be pulled against a second end stop **618₂** by spring **620.**

In an embodiment, a servomotor may be used to drive the switching body. Servomotors may be very accurate, and therefore, the end stops **618₁₋₂** and the spring **620** may be excluded.

In an embodiment, the switching body **602** may be attached directly to an axis **604** of a motor, e.g., a servomotor.

**Fig. 6B** schematically depicts a driving mechanism for moving a switching body **630** of an optical switch between a first position and a second position using a cam. Although the depicted switching body corresponds to an optical switch as depicted in **fig. 5C****,** the same driving mechanism may be used to move any suitable switching body, e.g., one as depicted in **fig. 5D****.**

The switching body **630** may comprise a first light path **632** for guiding light from a coherent light input to a light output **636** when the switching body is in the second position (depicted). The light path may comprise e.g., a mirror **634.** Alternatively, the light path may comprise e.g., an optical fibre. In the second position, part of the switching body may block light coming from an incoherent light input **638.** The switching body may further comprise a light guide for guiding incoherent light from the incoherent light input to the light input when the switching body is in the second position (the relative position of the incoherent light input and the light input with respect to the light guide has been shown using dashes).

A shaft **640** may be connected to the switching body **630.** An actuator **644** may be configured for moving cam **642.** By moving the cam in a first direction, e.g., parallel to a longitudinal axis of the switching body, the switching body may be moved in a second direction, e.g., a lateral direction perpendicular to the first direction. The cam may be shaped with high accuracy, thus ensuring accurate positioning of the switching body. The positioning may further be improved by a spring **646,** in particular in the first state.

**Fig. 6C** schematically depicts part of an optical switch comprising a rotating shutter. The shutter **650** may comprise one or more shutter blades **652** attached to a rotatable axis **654.** The shutter may be placed in the optical switch as described above with reference to **fig. 5A** and **5B****.** When the shutter rotates around the axis, the shutter blades may alternately obstruct and leave unobstructed an incoherent light or coherent light optical beam path **656.**

The optical switch may further comprise a light sensor **658** positioned on one side of the shutter and a light emitter positioned on the other side of the shutter, together forming an optical sensor arrangement substantially parallel with the rotation axis. In the depicted embodiment, the optical sensor arrangement is configured such that the optical sensor may receive light from the light emitter when the optical beam path is not obstructed by the shutter blades and does not receive light when the optical beam path is obstructed by the shutter blades. Thus, the light sensor may provide a signal associated with the beam path being obstructed or unobstructed to a controller of the optical switch and/or to an interface.

Alternatively or additionally, an optical sensor arrangement may be positioned such that the optical sensor receives light when the optical beam path is obstructed and does not receive light when the optical beam path is unobstructed. Similar optical sensor arrangements may also be included in other embodiments, e.g., in the embodiments depicted in **fig. 6A** and **6B****.**

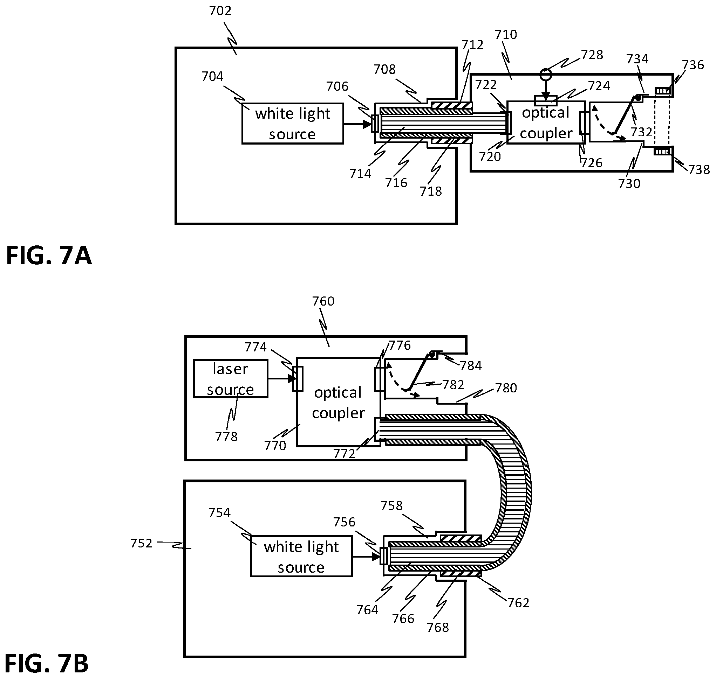

**Fig. 7A** and **7B** schematically depict connections between a light source unit of an endoscopic system and a coupling device according to an embodiment of the invention. **Fig. 7A** depicts a light source unit **702** of an endoscopic system comprising an incoherent light source **704,** e.g., a white light source, and an optical output **706** for providing light to an optical output connector **708.** The optical output connector is shaped to receive a connector from a light delivery system. Different makes or brands may have differently shaped optical output connectors.

**Fig. 7A** further depicts a coupling device **710** for injecting coherent light into the light delivery system of the endoscopic system. The coupling device comprises an optical input connector **712** for connecting to the optical output connector **708** of the light source unit **702.** In the depicted embodiment, the optical input connector comprises a rigid light pipe **714** for guiding light from the light source unit's optical output **706** to an optical coupler **720.** The optical input connector may further comprise a shaft **716,** preferably an opaque shaft, for protecting the light guide and preventing unwanted light from entering the light pipe. The shaft may cover the entire length of the light guide or parts of it, e.g., only exposed parts. The optical input connector may also comprise a holder part **718** for securing the light pipe to the light source unit's optical output connector **708.** The holder part may extend over a part of all of the length of the light pipe and/or of the connector to provide mechanical support and protection of the light pipe. A rigid light pipe allows for a connection with little intensity loss to the incoherent light and limited changes in spectrum.

Preferably, the shape and dimensions of the optical input connector **712** are selected to be compatible with a predetermined type of optical output connector **708.** In some embodiments, the optical input connector may be exchangeable or adjustable in order for the coupling device **710** to be compatible with a plurality of optical output connector types.

The optical coupler **720** may comprise an incoherent light input **722** for receiving incoherent light from the optical input connector. The optical coupler may further comprise a coherent light input **724** and a light output **726.** The coherent light input may receive light from a coherent light source directly or via an coherent light input connector **728.** It is an advantage of receiving coherent light via a coherent light connector that the coupling device can be relatively small and lightweight, allowing for the coupling device to be attached to the front of the light source unit **702** also when the light source unit is mounted in an optical tower, without requiring e.g., a dedicated external support structure, and without becoming cumbersome to the user by extending far. The optical coupler may be an optical switch as discussed above with reference to e.g., **fig. 5A** through **5D****,** preferably an optical switch as shown in **fig. 5A** through **5C****.**

The coupling device **710** may further comprise an optical output connector **730** for connecting to and providing light to the endoscopic system's the light delivery system. Preferably, the optical output connector is shaped and dimensioned to be compatible with the same type of optical connector as the coupling device's optical input connector **712.** This way, the coupling device may be connected to a light delivery system that is also connectable directly to the light source unit **702.**

As the coupling device **710** provides coherent light at its optical output **726,** the coupling device's optical output connector **730** may advantageously be equipped with one or more laser safety features **732-740.** For example, the optical output connector may comprise a shutter **732** for blocking light, in particular coherent light, from exiting the optical output connector **730** if there is no light delivery system connected to the optical output connector **730.** The shutter may be pushed to a shut position by e.g., a spring **734** when the output connector is empty, and may be pushed to an open position when an input connector is inserted. Alternatively or additionally, the optical output connector may comprise an optical sensor **736,738.** The optical sensor may comprise a light source **736** and a sensor **738.** The sensor may provide a first signal to a controller of the coupling device if more than a predetermined amount of light from the light source **736** is detected by the sensor **738,** and a second signal if less than the predetermined amount is detected. When the controller receives the first signal, the controller may, depending on the embodiment, e.g., switch off the first and/or second laser sources, and/or block the coherent light path by switching to a state in which the coherent light is not injected to the optical output connector.

In the depicted embodiment, the light guide **714** from the optical input connector **712** connects directly to the optical coupler **720.** An advantage of this configuration is its constructional simplicity and low loss in light intensity. However, in other embodiments, there may be intermediate structures between the optical input connector and the optical coupler. Similarly, the light output **726** of the optical coupler is connected directly to the optical output connector **730.** This is again advantageous for the light intensity and quality of the output light. Again, in other embodiments, there may be intermediate structures to guide light from the light output **726** to the optical output connector **730.**

**Fig. 7B** depicts an alternative type of connection between a light source unit **752** of an endoscopic system and a coupling device **760.** The light source unit may comprise an incoherent light source **754,** e.g., a white light source, an optical output **756** and an optical output connector **758** as discussed above with reference to **fig. 7A****.**

The coupling device **760** may comprise an optical input connector **762** comprising a shaft **766** and a holder **768** as discussed above with reference to **fig. 7A****.** The input connector also comprises a light guide **764,** but in this embodiment, the light guide is a flexible light guide, e.g., a fibre bundle, a fibre bundle with fused ends, or a liquid light guide. This allows for flexible placement of the coupling device, e.g., on a different platform of an optical tower housing the light source unit. As a result, there are less restrictions to e.g., size and weight, compared to a situation where the coupling device is appended to a front of the light source unit, facilitating incorporation of one or more coherent light sources **778** in the coupling device. This has an advantageous effect on the light quality of the coherent light, as the coherent light can travel almost entirely through free space without safety concerns.

The coupling device **760** further comprises an optical coupler **770** comprising a incoherent light input **772,** a coherent light input **774** and a light output **776.** The optical coupler may be an optical switch as discussed above with reference to e.g., **fig. 5A** through **5D****,** preferably an optical switch as shown in **fig. 5D****.** If the optical coupler is an optical switch as shown in **fig. 5D****,** the flexible light guide **764** may be the same as the flexible light guide **594,** thus minimising transitions and maximising light quality.

The coupling device **760** may further comprise an optical output connector **780** and safety features, e.g., a shutter **782** operated by a spring **784** and/or an optical sensor (not depicted), as discussed above with reference to **fig. 7A****.**

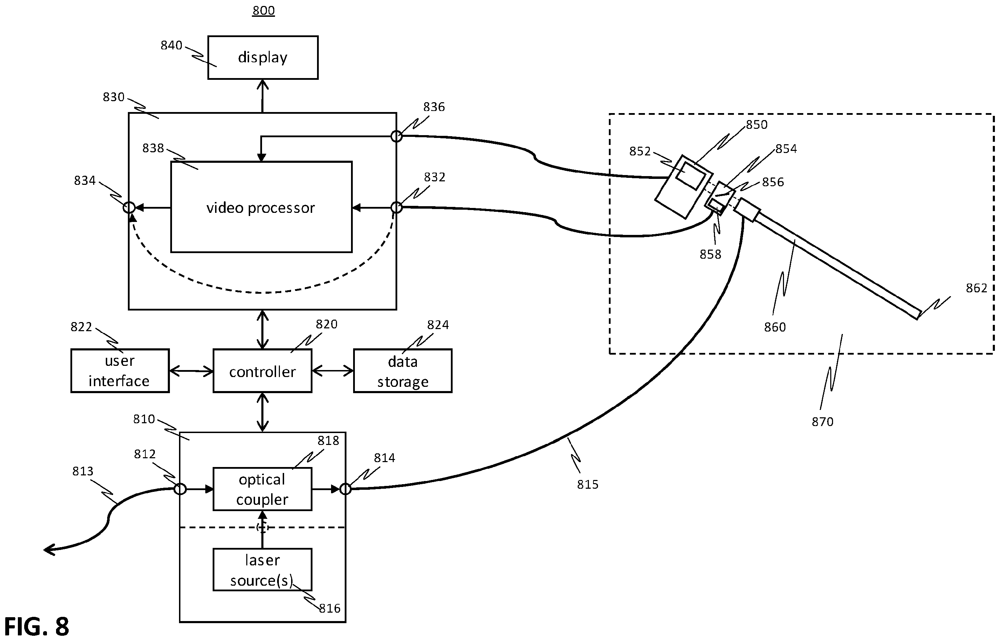

**Fig. 8** schematically depicts a coherent light system for adding coherent light imaging capability to an endoscopic system according to an embodiment of the invention. The coherent light system **800** comprises a coupling device **810** comprising an optical input **812** for receiving incoherent light from a light source of the endoscopic system via a light guide **813,** an optical output **814** for providing light to a light guide **815** of a light delivery system of the endoscopic system, and one or more coherent light sources **816** for generating light of at least a first wavelength. The coupling device further comprises an optical coupler **818** for alternately providing the incoherent light and the coherent light to the light delivery system.

Optionally, the coherent light system **800** comprises a controller **820,** operatively connectable to the optical coupler **818,** for controlling the optical coupler. The controller may be included in the same device as the optical coupler and/or as the one or more laser sources, or may be embodied in a separate controller device and configured to communicate with the coupling device **810** using a wired or wireless connection.

Optionally, the coherent light system **800** may comprise a user interface **822** connectable to the controller **820** for receiving input for controlling the system and/or providing output regarding the status of the system. The user interface may comprise one or more buttons, e.g., a power button and a button for generating trigger signal. The user interface may also comprise one or more status lights. The user interface may be included in the same device as the controller and/or the optical coupler **818.** Alternatively, the user interface may be embodied in a different device, e.g., a handheld device, configured to communicate via a wired or wireless connection with the controller and/or the coupling device **810.** The user interface may also be distributed over several components of the system, e.g., a button attached to a handle of an endoscope **870** configured to send a trigger signal to the coupling device, and a status light on the coupling device indicating the status of the optical coupler **818.** The status of the optical coupler is particular relevant when the optical coupler is an optical switch.

Optionally, the coherent light system **800** may comprise or be connectable to an internal or external data storage **824** for storing information related to the status of the coupling device and/or for storing images acquired with the endoscopic system, if the system comprises a video processing module **830.** The data storage may be operatively connectable to the controller **820** via a wired or wireless connection.

Optionally, the coherent light system **800** may comprise a video processing module **830.** The video processing module may comprise a first video input connector **832** for receiving a first video stream from a camera for capturing endoscopic images, the camera preferably being included in or attached to the endoscope **870.** The video processing module may further comprise a video output connector **834** for providing the video input stream or a derivative thereof to a video processing module of the endoscopic system. In some embodiments, the video processing module may comprise a second video input connector **836** for receiving a second video stream from a camera for capturing endoscopic images. The video processing module may further comprise a video processor **838** for processing the first and, optionally, second video streams. Preferably, the video processor comprises a dedicated graphics processor for processing the video stream. The video processor may further comprise a general processing unit and a memory for storing software, e.g., image processing software for generating laser speckle contrast images and/or fluorescence images. The video processing module may further comprise e.g., a frame grabber, a video converter, and a buffer.

The image processor may comprise a computer-readable storage medium having computer-readable program code embodied therewith, and a processor, preferably a microprocessor, coupled to the computer-readable storage medium. The computer-readable program code may comprise instructions to execute method steps for determining a coherent light image, preferably a fluorescence image such as an ICG-based image or a laser speckle contrast image.

The video processing module may be implemented as a cloud-based module. In such an embodiment, the video processing module may comprise a network connection for connecting to the Internet, and the image processor may be an Internet-connected computing device.

In an embodiment, the video processor may process the first and/or second video streams in dependence on the status of the optical coupler. For example, if the optical coupler is an optical switch, the video processor may be configured or controlled by the controller **820** to only process the one or more video streams when the optical switch is in a state in which the coherent light is provided to the light delivery system.

The video processing module **830** may be included in the same device as the controller **820,** or may be embodied in a separate image processing device and configured to communicate with the controller using a wired or wireless connection. The video processor and the controller may also be embodied as software modules running on shared hardware, e.g., an embedded computing module comprising a central processing unit (CPU), a memory, and, optionally, a graphics processing unit (GPU).

The coherent light system **800** may further comprise a display **840,** connectable to the video processing module **830.** The system may thus be configured to display images or video based on coherent light imaging on the display **840,** while images or video based on incoherent light imaging are displayed on display included in the endoscopic system.

The endoscopic system comprises an endoscope **870.** The endoscope may comprise a flexible or rigid insertion tube **860** with a distal tip **862** for inserting into an object, preferably a patient, e.g., a body of a human or an animal, preferably a living body. Some endoscopic systems, typically systems with a flexible insertion tube, may have an image sensor mounted at or near the distal tip **862.** Other endoscopic systems, typically systems with a rigid insertion tube, may have an imaging unit comprising an image sensor, e.g., an RGB camera, located in or near a handle of the endoscope. In some systems, the imaging unit is detachable from the insertion tube.

Optionally, the coherent light system **800** may comprise an imaging unit **850** configured to be attached to a proximal end of an insertion tube of an endoscope. The imaging unit may comprise a camera, e.g., an RGB or an RGB/IR camera **852.** An advantage of an RGB/IR camera is that infrared coherent light may be acquired simultaneously with incoherent light images or white light image. This way, images obtained using coherent light and images based on the incoherent light may be combined in an relatively easy manner, without requiring e.g., alignment of the images based on coherent and incoherent light. If the first wavelength is in the visible spectrum, coherent light images may be obtained by reading the respective channels of the RGB camera, e.g., if the first wavelength is in the red part of the electromagnetic spectrum, a coherent light image may be obtained by reading or using only the red pixels of an RGB image captured during illumination of a target with red coherent light and preferably without simultaneous illumination with incoherent light.

Alternatively, the coherent light system **800** may comprise a dedicated imaging module **854** for acquiring images of light at the first wavelength. The dedicated imaging module may comprise a beam splitter **856,** e.g., a dichroic mirror or polarising beam splitter, for splitting light collected by the endoscope in a first light bundle, comprising predominantly light of approximately the first wavelength or light of a wavelength emitted by a fluorescent marker, and a second light bundle, comprising the remainder of the light. The dedicated imaging module may further comprise a dedicated image sensor **858** for imaging the first light bundle.

The dedicated imaging module **854** may be configured to be attached to a proximal end of the **860** insertion tube of the endoscope **870.** The dedicated imaging module may further be configured to receive an imaging unit **850** that is configured to be attached to the proximal end of the insertion tube of the endoscope. This way, dedicated imaging, e.g., infrared imaging, may be added to an endoscope with a detachable camera. If the first wavelength is in the infrared part of the electromagnetic spectrum, an endoscope without an infrared filter in the insertion tube or connection point should be selected.

In some embodiments of the coherent light system **800,** the optical coupler **818** may be configured as an optical switch capable of switching at a high frequency, e.g., 60 Hz. In such an embodiment, the imaging unit **850** may be an RGB camera configured to acquire images or a video stream at a high framerate, e.g., 120 frames per second. The camera may be operatively connected to the controller 820. Thus, the coherent light system is configured to alternately capture a coherent light image and an incoherent light image, e.g., a white light image. The video processing module **830** may, for example, output a video stream based on the incoherent light images with a framerate of 60 fps at the video signal output **834,** and may send a video stream based on the coherent light images to display **840,** also with a framerate of 60 fps. In some embodiments, images acquired when the optical switch was switching between a first state and a second state may be of lower quality. In that case, the camera may e.g., acquire a video stream comprising sequences of an incoherent light image, a switching image, a coherent light image, and another switching image. The video processing module may disregard the switching images, and generate video streams based on coherent light and on incoherent light at 30 fps each.

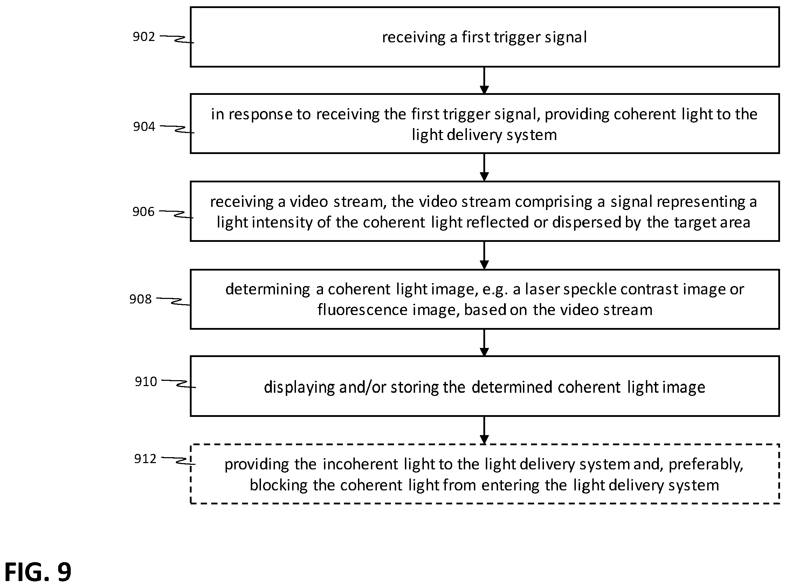

**Fig. 9** depicts a method for generating a coherent light image using a coherent light coupling system according to an embodiment of the invention. The coherent light coupling system may comprise an optical input for receiving incoherent light from a light source of an endoscopic system, a first coherent light source for generating coherent light of a first wavelength, and an optical output for alternately providing the incoherent light and the coherent light of the first wavelength to a light delivery system of the endoscopic system. The optical output may be connect to the endoscope via a first light guide and the optical input may be connected to the incoherent light source via a second light guide.

The coherent light coupling system further comprises an optical switch configured for switching between a first state wherein the incoherent light is injected into the optical output and the coherent light is not injected into the optical output and, hence, not into the first light guide, and a second state wherein the coherent light is injected into the optical output and the incoherent light is not injected into the optical output and, hence, into the first light guide.

In a first step 902, a controller in the coherent light coupling system receives a first trigger signal. In response to receiving the first trigger signal, the controller causes the optical switch to switch **904** to the second state. Thus, coherent light may be provided to the light delivery system of the endoscopic system. Consequently, the endoscope may illuminate a target area with coherent light, and collect light reflected or emitted by tissue in the target area in response to being illuminated. A camera of the endoscopic system may capture a video stream based on the collected light.

In a next step **906,** a video processing module in the coherent light coupling system may receive the video stream from the camera of the endoscopic system, the video stream comprising image information based on illuminating the target area with coherent light of the first wavelength. Subsequently, the video processing module may determine 908 one or more coherent light images, e.g., laser speckle contrast images or fluorescence images such as ICG-based images, based on the received video stream. In an embodiment, the video stream may comprise frames or the video processing module may be configured to determine frames based on the received video stream. A single coherent light image may be based on a plurality of frames of the received video stream.

In a next step **910,** the coherent light coupling system may provide the one or more determined coherent light images to a video signal output of the coherent light coupling system, display the one or more determined coherent light images on a display of the coherent light coupling system and/or store the one or more determined coherent light images in a database included in or connected to the coherent light coupling system.

In an optional step **912,** the coherent light coupling system may cause the optical switch to switch to the first state after a predetermined number of coherent light images has been determined, after a predetermined amount of time, or upon receiving a second trigger signal.

The terminology used herein is for the purpose of describing particular embodiments only and is not intended to be limiting of the invention. As used herein, the singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements in the claims below are intended to include any structure, material, or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope of the invention. The embodiment was chosen and described in order to best explain the principles of the invention and the practical application, and to enable others of ordinary skill in the art to understand the invention for various embodiments with various modifications as are suited to the particular use contemplated.

## Claims

1. A coupling device (140) for coupling coherent light and incoherent light into a first light guide (124) of a light delivery system of an endoscopic system (100_{1,2}), the endoscopic system (100_{1,2}) comprising an endoscope (106) and an incoherent light source (108) for generating the incoherent light, e.g., white light, the first light guide (124) being configured to connect the coupling device (140) to the endoscope (106), the coupling device (140) comprising:
an optical input (142) comprising an optical input connector for releasably connecting the coupling device (140) to the incoherent light source (108) via a second light guide (158), the optical input (142) being configured for receiving the incoherent light from the endoscopic system (100_{1,2});
a first light source (146) for generating coherent light of a first wavelength;
an optical output (144) comprising an optical output connector for releasably connecting the coupling device (140) to the endoscope (106) via the first light guide (124), the optical output (144) being configured for providing the incoherent light and the coherent light of the first wavelength to the first light guide (124);
an optical switch (148) for alternately injecting the incoherent light and the coherent light of the first wavelength into the first light guide (124), wherein the coherent light and the incoherent light have a shared optical path at the optical output (144); and
a control switch for generating a trigger signal or a trigger input for receiving a trigger signal, wherein the optical switch is configured to switch from a first state to a second state in response to receiving a trigger signal;
wherein in at least the first state of the coupling device (140), the coupling device (140) comprises an unobstructed incoherent light beam path for propagating the incoherent light from the optical input (142) to the optical output (144), and the coherent light is not injected into the first light guide (124); and
wherein in at least the second state of the coupling device (140), the coupling device (140) comprises an unobstructed coherent light beam path for propagating the coherent light from the coherent light source (146) to the optical output (144) and the coherent light is injected into the first light guide (124).

2. The coupling device as claimed in claim 1, wherein in the first state, the optical switch is configured to inject the incoherent light into the first light guide, and wherein in the second state, the optical switch is configured not to inject the incoherent light into the first light guide.

3. The coupling device as claimed in claim 1 or 2, wherein the optical switch comprises:
a first mirror movable between a first position in which a reflective surface of the first mirror is positioned to reflect incoherent light propagating over the incoherent light beam path, and a second position, in which the reflective surface of the first mirror is positioned to reflect the incoherent light in a direction away from the incoherent light beam path; and/or a second mirror movable between a first position in which a reflective surface of the second mirror is positioned to reflect coherent light propagating over the coherent light beam path, and a second position, in which the reflective surface of the first mirror is positioned to reflect the coherent light in a direction away from the coherent light beam path.

4. The coupling device as claimed in claim 3, wherein the optical switch is an electrically operated switch with a motor, optionally a servomotor; the motor being configured to move the first mirror and, optionally, the second mirror, between the first position and the second position.

5. The coupling device as claimed in claim 1 or 2, wherein the optical switch comprises:
a first switchable mirror, switchable between a transparent state and a reflective state, positioned to reflect, in the reflective state, incoherent light propagating over the incoherent light beam path; and/or a second switchable mirror, switchable between a transparent state and a reflective state, positioned to reflect, in the reflective state, coherent light propagating over the coherent light beam path;
or wherein the optical switch comprises:
a first light beam blocker configured to block the coherent light in the first state, preferably the first beam blocker comprising a mechanical shutter, preferably a rotating shutter, more preferably a motorised rotating shutter, or an optical beam blocker, preferably one of: a liquid crystal light valve, an acousto-optical modulator, an electro-optical modulator or a photo-elastic modulator; and, optionally, a second light beam blocker configured to block the incoherent light in the second state, preferably the second beam blocker comprising a mechanical shutter, preferably a rotating shutter, more preferably a motorised rotating shutter, or an optical beam blocker, preferably a liquid crystal light valve.

6. The coupling device as claimed in any of the preceding claims, wherein the optical switch comprises:
a first convergent lens to reduce divergence of the incoherent light from the optical input and a second convergent lens to focus the incoherent light into the first light guide; and
a mirror, positioned between the first lens and the second lens, configured to steer the coherent light beam and/or the incoherent light beam into the first light guide.

7. The coupling device as claimed in any of the preceding claims, wherein the optical switch comprises:
a tapered light pipe having a wide end for receiving the incoherent light from the optical input and a narrow end for providing the incoherent light to the optical output, the tapered light pipe being divided into a first part and a second part by a plane intersecting the tapered light pipe between the wide end and the narrow end, a normal of the plane defining a non-zero angle with a longitudinal axis of the tapered light pipe, preferably, the tapered light pipe further comprising a protrusion having a surface normal to the coherent light path for receiving the coherent light; and
a mirror, positioned on the plane between the first part and the second part of the tapered light pipe on the longitudinal axis of the tapered light pipe, configured to steer the laser beam into the first light guide.

8. The coupling device as claimed in claim 1 or 2, wherein the optical switch comprises:
an internal light guide for guiding the incoherent light from a first end of the internal light guide to a second end of the internal light guide; and
a switching body comprising at least the second end of the internal light guide, the switching body being movable between a first position corresponding to the first state and a second position corresponding to the second state,
- in which first position, the switching body is configured to block the coherent light beam path, to position the first end of the internal light guide for receiving incoherent light from the optical input, and to position the second end for injecting the incoherent light into the first light guide; and
- in which second position, the switching body is configured not to block the coherent light beam path, and to position at least the second end of the internal light guide such that the incoherent light is not injected into the first light guide.

9. The coupling device as claimed in claim 8, wherein the internal light guide is a flexible light guide, preferably a fused fibre bundle or a liquid light guide, and wherein the first end of the internal light guide is connected to the optical input.

10. The coupling device as claimed in claim 8 or 9, wherein the optical switch is an electrically operated switch with a motor, optionally a servomotor; the motor being configured to move the switching body between the first position and the second position via a cam or a crank.

11. The coupling device as claimed in any one of the preceding claims,
wherein the second light guide is a flexible light guide, preferably a fibre bundle, a fused fibre bundle, or a liquid light guide.

12. The coupling device as claimed in any of the preceding claims, wherein the first light source is a narrow-bandwidth laser, preferably having a spectrum bandwidth of less than 1 nm, more preferably less than 0.2 nm, even more preferably less than 0.1 nm.

13. The coupling device as claimed in any of the preceding claims, wherein the first light source is a laser with a power output of at least 20 mW, preferably at least 100 mW, even more preferably more at least 150 mW.

14. The coupling device as claimed in any of the preceding claims, wherein the first wavelength is in the red part of the electromagnetic spectrum, preferably in the range 600-700 nm, more preferably in the range 630-660 nm, or wherein the first wavelength is in the infrared part of the electromagnetic spectrum, preferably in the range 700-1200 nm, more preferably in the range 700-900 nm, even more preferably in the range 770-790 nm or in the range 820-840 nm.

15. The coupling device as claimed in any of the preceding claims, wherein the endoscopic system comprises a camera, preferably an RGB camera, the camera being configured to provide a video signal, and wherein the coupling device further comprises:
a video signal input connector for receiving the video signal;
a video signal output connector for providing a video output; and
an image processing module for generating coherent light images, preferably fluorescence images and/or laser speckle contrast images based on the video signal when the optical switch injects coherent light into the first light guide.

16. The coupling device as claimed in claim 15, wherein
in the first state, the video signal is looped to the video output; and
wherein in the second state, the video signal is provided to the image processing module, the image processing module being configured to determine one or more output images, preferably laser speckle contrast images or fluorescence images, based on the video signal, and the one or more output images being provided at the video signal output connector.

17. The coupling device as claimed in any of the preceding claims, further comprising a first sensor, preferably an optical sensor, and a controller, connected to the first sensor, wherein:
the first sensor is configured to send a first signal to the controller only if the first light guide is connected to the optical output connector; and
the controller is configured to switch off or block the first coherent light source, if the controller does not receive the first signal.

18. An image processing system for generating coherent light images with an endoscopic system, the endoscopic system comprising an incoherent light source, a video processing unit, and an endoscope, the endoscope being connected to a camera, the image processing system comprising a coupling device as claimed in any of claims 1-12 and a video processing device, the video processing device comprising:
- a video signal input connector for receiving a video signal from the camera;
- a first video signal output connector for providing a video output, the first video signal output connector configured to be connected to a video signal input of the video processing unit of the endoscopic system; and
- an image processing module for generating coherent light images based the video signal when the optical switch is in the first state.

19. An imaging system for generating an infrared coherent light image with an endoscopic system, the endoscopic system comprising an incoherent light source, an endoscope, and a light delivery system for delivering light to the endoscope, the imaging system comprising:
a coupling device as claimed in any of claims 1-11, the first wavelength being selected in the infrared part of the electromagnetic system, preferably in the range 700-1200 nm, more preferably in the range 700-900 nm, even more preferably in the range 770-790 nm or in the range 820-840 nm;
an infrared imaging sensor, configured to receive light collected by the endoscope; and
an image processing module, configured to receive a video signal from the infrared imaging sensor and to determine a coherent light image based on the received video signal.
preferably an RGB/IR camera or a beam splitter for splitting the light reflected by the target into an IR beam and a white light beam and an IR camera.

20. An endoscopic system, preferably a laparoscopic system, comprising the coupling device as claimed in any of claims 1-17.

## Patentansprüche

1. Kopplungsvorrichtung (140) zum Einkoppeln von kohärentem Licht und inkohärentem Licht in einen ersten Lichtleiter (124) eines Lichtzuführungssystems eines endoskopischen Systems (100_{1,2}), wobei das endoskopische System (100_{1,2}) ein Endoskop (106) und eine inkohärente Lichtquelle (108) zum Erzeugen des inkohärenten Lichts, z.B. weißes Licht, aufweist, wobei der erste Lichtleiter (124) dazu konfiguriert ist, die Kopplungsvorrichtung (140) mit dem Endoskop (106) zu verbinden, wobei die Kopplungsvorrichtung (140) aufweist:
einen optischen Eingang (142), der einen optischen Eingangsanschluss zum lösbaren Verbinden der Kopplungsvorrichtung (140) mit der inkohärenten Lichtquelle (108) über einen zweiten Lichtleiter (158) aufweist, wobei der optische Eingang (142) zum Empfangen des inkohärenten Lichts von dem endoskopischen System(100_{1,2}) konfiguriert ist;
eine erste Lichtquelle (146) zum Erzeugen von kohärentem Licht einer ersten Wellenlänge;
einen optischen Ausgang (144), der einen optischen Ausgangsanschluss zum lösbaren Verbinden der Kopplungsvorrichtung (140) mit dem Endoskop (106) über den ersten Lichtleiter (124) aufweist, wobei der optische Ausgang (144) dazu konfiguriert ist, das inkohärente Licht und das kohärente Licht der ersten Wellenlänge dem ersten Lichtleiter (124) bereitzustellen;
einen optischen Schalter (148) zum abwechselnden Einkoppeln des inkohärenten Lichts und des kohärenten Lichts der ersten Wellenlänge in den ersten Lichtleiter (124), wobei das kohärente Licht und das inkohärente Licht einen gemeinsamen optischen Pfad am optischen Ausgang (144) haben; und
einen Steuerschalter zum Erzeugen eines Triggersignals oder einen Triggereingang zum Empfangen eines Triggersignals, wobei der optische Schalter dazu konfiguriert ist, als Reaktion auf den Empfang eines Triggersignals von einem ersten Zustand in einen zweiten Zustand zu schalten;
wobei in mindestens dem ersten Zustand der Kopplungsvorrichtung (140) die Kopplungsvorrichtung (140) einen unbehinderten Strahlengang für inkohärentes Licht zur Ausbreitung des inkohärenten Lichts vom optischen Eingang (142) zum optischen Ausgang (144) aufweist, und das kohärente Licht nicht in den ersten Lichtleiter (124) eingekoppelt wird; und
wobei in mindestens dem zweiten Zustand der Kopplungsvorrichtung (140) die Kopplungsvorrichtung (140) einen unbehinderten Strahlengang für kohärentes Licht zur Ausbreitung des kohärenten Lichts von der ersten Lichtquelle (146) zum optischen Ausgang (144) aufweist und das kohärente Licht in den ersten Lichtleiter (124) eingekoppelt wird.

2. Kopplungsvorrichtung nach Anspruch 1, wobei im ersten Zustand der optische Schalter dazu konfiguriert ist, das inkohärente Licht in den ersten Lichtleiter einzukoppeln, und wobei im zweiten Zustand der optische Schalter dazu konfiguriert ist, das inkohärente Licht nicht in den ersten Lichtleiter einzukoppeln.

3. Kopplungsvorrichtung nach Anspruch 1 oder 2, wobei der optische Schalter aufweist:
einen ersten Spiegel, der zwischen einer ersten Position, in der eine reflektierende Oberfläche des ersten Spiegels so positioniert ist, dass sie inkohärentes Licht reflektiert, das sich über den Strahlengang für inkohärentes Licht ausbreitet, und einer zweiten Position beweglich ist, in der die reflektierende Oberfläche des ersten Spiegels so positioniert ist, dass sie das inkohärente Licht in eine Richtung weg vom Strahlengang für inkohärentes Licht reflektiert; und/oder einen zweiten Spiegel, der zwischen einer ersten Position, in der eine reflektierende Oberfläche des zweiten Spiegels so positioniert ist, dass sie kohärentes Licht reflektiert, das sich über den Strahlengang für kohärentes Licht ausbreitet, und einer zweiten Position beweglich ist, in der die reflektierende Oberfläche des ersten Spiegels so positioniert ist, dass sie das kohärente Licht in eine Richtung weg vom Strahlengang für kohärentes Licht reflektiert.

4. Kopplungsvorrichtung nach Anspruch 3, wobei der optische Schalter ein elektrisch betriebener Schalter mit einem Motor, optional einem Servomotor, ist; wobei der Motor dazu konfiguriert ist, den ersten Spiegel und, optional, den zweiten Spiegel zwischen der ersten Position und der zweiten Position zu bewegen.

5. Kopplungsvorrichtung nach Anspruch 1 oder 2, wobei der optische Schalter aufweist:
einen ersten schaltbaren Spiegel, der zwischen einem transparenten Zustand und einem reflektierenden Zustand schaltbar ist, positioniert, um im reflektierenden Zustand inkohärentes Licht zu reflektieren, das sich über den Strahlengang für inkohärentes Licht ausbreitet; und/oder einen zweiten schaltbaren Spiegel, der zwischen einem transparenten Zustand und einem reflektierenden Zustand schaltbar ist, positioniert, um im reflektierenden Zustand kohärentes Licht zu reflektieren, das sich über den Strahlengang für kohärentes Licht ausbreitet;
oder wobei der optische Schalter aufweist:
einen ersten Lichtstrahlblocker, der dazu konfiguriert ist, das kohärente Licht im ersten Zustand zu blockieren, wobei der erste Lichtstrahlblocker vorzugsweise einen mechanischen Verschluss, vorzugsweise einen rotierenden Verschluss, noch bevorzugter einen motorisierten rotierenden Verschluss, oder einen optischen Lichtstrahlblocker, vorzugsweise einen von: einem Flüssigkristall-Lichtventil, einem akusto-optischen Modulator, einem elektro-optischen Modulator oder einem photoelastischen Modulator, aufweist; und, optional, einen zweiten Lichtstrahlblocker, der dazu konfiguriert ist, das inkohärente Licht im zweiten Zustand zu blockieren, wobei der zweite Lichtstrahlblocker vorzugsweise einen mechanischen Verschluss, vorzugsweise einen rotierenden Verschluss, noch bevorzugter einen motorisierten rotierenden Verschluss, oder einen optischen Lichtstrahlblocker, vorzugsweise ein Flüssigkristall-Lichtventil, aufweist.

6. Kopplungsvorrichtung nach einem der vorhergehenden Ansprüche, wobei der optische Schalter aufweist:
eine erste Sammellinse zur Verringerung der Divergenz des inkohärenten Lichts vom optischen Eingang und eine zweite Sammellinse zur Fokussierung des inkohärenten Lichts in den ersten Lichtleiter; und
einen Spiegel, der zwischen der ersten Linse und der zweiten Linse positioniert ist und dazu konfiguriert ist, den kohärenten Lichtstrahl und/oder den inkohärenten Lichtstrahl in den ersten Lichtleiter zu lenken.

7. Kopplungsvorrichtung nach einem der vorhergehenden Ansprüche, wobei der optische Schalter aufweist:
ein konisches Lichtrohr mit einem weiten Ende zum Empfangen des inkohärenten Lichts vom optischen Eingang und einem engen Ende zum Bereitstellen des inkohärenten Lichts an den optischen Ausgang, wobei das konische Lichtrohr durch eine Ebene, die das konische Lichtrohr zwischen dem weiten Ende und dem engen Ende schneidet, in einen ersten Teil und einen zweiten Teil geteilt ist, wobei eine Normale der Ebene einen von Null verschiedenen Winkel mit einer Längsachse des konischen Lichtrohrs definiert, wobei das konische Lichtrohr vorzugsweise ferner einen Vorsprung mit einer zur kohärenten Lichtbahn normalen Oberfläche zum Empfangen des kohärenten Lichts aufweist; und
einen Spiegel, der auf der Ebene zwischen dem ersten Teil und dem zweiten Teil des konischen Lichtrohrs auf der Längsachse des konischen Lichtrohrs positioniert ist und dazu konfiguriert ist, den Laserstrahl in den ersten Lichtleiter zu lenken.

8. Kopplungsvorrichtung nach Anspruch 1 oder 2, wobei der optische Schalter aufweist:
einen internen Lichtleiter zum Führen des inkohärenten Lichts von einem ersten Ende des internen Lichtleiters zu einem zweiten Ende des internen Lichtleiters; und
einen Schaltkörper, der mindestens das zweite Ende des internen Lichtleiters aufweist, wobei der Schaltkörper zwischen einer ersten Position, die dem ersten Zustand entspricht, und einer zweiten Position, die dem zweiten Zustand entspricht, beweglich ist,
- wobei in der ersten Position der Schaltkörper dazu konfiguriert ist, den kohärenten Strahlengang zu blockieren, das erste Ende des internen Lichtleiters zum Empfangen von inkohärentem Licht vom optischen Eingang zu positionieren und das zweite Ende zum Einkoppeln des inkohärenten Lichts in den ersten Lichtleiter zu positionieren; und
- wobei in der zweiten Position der Schaltkörper dazu konfiguriert ist, den kohärenten Strahlengang nicht zu blockieren und mindestens das zweite Ende des internen Lichtleiters so zu positionieren, derart, dass das inkohärente Licht nicht in den ersten Lichtleiter eingekoppelt wird.

9. Kopplungsvorrichtung nach Anspruch 8, wobei der interne Lichtleiter ein flexibler Lichtleiter ist, vorzugsweise ein Faserbündel oder ein Flüssiglichtleiter, und wobei das erste Ende des internen Lichtleiters mit dem optischen Eingang verbunden ist.

10. Kopplungsvorrichtung nach Anspruch 8 oder 9, wobei der optische Schalter ein elektrisch betriebener Schalter mit einem Motor, optional einem Servomotor, ist; wobei der Motor dazu konfiguriert ist, den Schaltkörper zwischen der ersten Position und der zweiten Position mittels einer Nocke oder einer Kurbel zu bewegen.

11. Kopplungsvorrichtung nach einem der vorhergehenden Ansprüche, wobei der zweite Lichtleiter ein flexibler Lichtleiter ist, vorzugsweise ein Faserbündel, ein verschmolzenes Faserbündel oder ein Flüssiglichtleiter.

12. Kopplungsvorrichtung nach einem der vorhergehenden Ansprüche, wobei die erste Lichtquelle ein Schmalbandlaser ist, der vorzugsweise eine spektrale Bandbreite von weniger als 1 nm, bevorzugter weniger als 0,2 nm, noch bevorzugter weniger als 0,1 nm aufweist.

13. Kopplungsvorrichtung nach einem der vorhergehenden Ansprüche, wobei die erste Lichtquelle ein Laser mit einer Ausgangsleistung von mindestens 20 mW, vorzugsweise mindestens 100 mW, noch bevorzugter mindestens 150 mW ist.

14. Kopplungsvorrichtung nach einem der vorhergehenden Ansprüche, wobei die erste Wellenlänge im roten Teil des elektromagnetischen Spektrums liegt, vorzugsweise im Bereich von 600-700 nm, bevorzugter im Bereich von 630-660 nm, oder wobei die erste Wellenlänge im infraroten Teil des elektromagnetischen Spektrums liegt, vorzugsweise im Bereich von 700-1200 nm, bevorzugter im Bereich von 700-900 nm, noch bevorzugter im Bereich von 770-790 nm oder im Bereich von 820-840 nm.

15. Kopplungsvorrichtung nach einem der vorhergehenden Ansprüche, wobei das endoskopische System eine Kamera, vorzugsweise eine RGB-Kamera, aufweist, wobei die Kamera dazu konfiguriert ist, ein Videosignal bereitzustellen, und wobei die Kopplungsvorrichtung ferner aufweist:
einen Videosignal-Eingangsanschluss zum Empfangen des Videosignals;
einen Videosignal-Ausgangsanschluss zum Bereitstellen eines Videoausgangs; und
ein Bildverarbeitungsmodul zum Erzeugen von Bildern mit kohärentem Licht, vorzugsweise Fluoreszenzbildern und/oder Laser-Speckle-Kontrastbildern, basierend auf dem Videosignal, wenn der optische Schalter kohärentes Licht in den ersten Lichtleiter einkoppelt.

16. Kopplungsvorrichtung nach Anspruch 15, wobei
im ersten Zustand das Videosignal zum Videoausgang durchgeschleift wird; und
wobei im zweiten Zustand das Videosignal dem Bildverarbeitungsmodul bereitgestellt wird, wobei das Bildverarbeitungsmodul dazu konfiguriert ist, ein oder mehrere Ausgangsbilder, vorzugsweise Laser-Speckle-Kontrastbilder oder Fluoreszenzbilder, basierend auf dem Videosignal zu bestimmen, und das eine oder die mehreren Ausgangsbilder am Videosignal-Ausgangsanschluss bereitgestellt werden.

17. Kopplungsvorrichtung nach einem der vorhergehenden Ansprüche, ferner aufweisend einen ersten Sensor, vorzugsweise einen optischen Sensor, und eine Steuerung, die mit dem ersten Sensor verbunden ist, wobei:
der erste Sensor dazu konfiguriert ist, ein erstes Signal nur dann an die Steuerung zu senden, wenn der erste Lichtleiter mit dem optischen Ausgangsanschluss verbunden ist; und
die Steuerung dazu konfiguriert ist, die erste kohärente Lichtquelle auszuschalten oder zu blockieren, wenn die Steuerung das erste Signal nicht empfängt.

18. Bildverarbeitungssystem zum Erzeugen von Bildern mit kohärentem Licht mit einem endoskopischen System, wobei das endoskopische System eine inkohärente Lichtquelle, eine Videoverarbeitungseinheit und ein Endoskop aufweist, wobei das Endoskop mit einer Kamera verbunden ist, wobei das Bildverarbeitungssystem eine Kopplungsvorrichtung nach einem der Ansprüche 1-12 und eine Videoverarbeitungsvorrichtung aufweist, wobei die Videoverarbeitungsvorrichtung aufweist:
- einen Videosignal-Eingangsanschluss zum Empfangen eines Videosignals von der Kamera;
- einen ersten Videosignal-Ausgangsanschluss zum Bereitstellen eines Videoausgangs, wobei der erste Videosignal-Ausgangsanschluss dazu konfiguriert ist, mit einem Videosignal-Eingang der Videoverarbeitungseinheit des endoskopischen Systems verbunden zu werden; und
- ein Bildverarbeitungsmodul zum Erzeugen von Bildern mit kohärentem Licht basierend auf dem Videosignal, wenn sich der optische Schalter im ersten Zustand befindet.

19. Bildgebungssystem zum Erzeugen eines Infrarotbildes mit kohärentem Licht mit einem endoskopischen System, wobei das endoskopische System eine inkohärente Lichtquelle, ein Endoskop und ein Lichtzuführungssystem zum Zuführen von Licht zum Endoskop aufweist, wobei das Bildgebungssystem aufweist:
eine Kopplungsvorrichtung nach einem der Ansprüche 1-11, wobei die erste Wellenlänge im infraroten Teil des elektromagnetischen Spektrums ausgewählt ist, vorzugsweise im Bereich von 700-1200 nm, bevorzugter im Bereich von 700-900 nm, noch bevorzugter im Bereich von 770-790 nm oder im Bereich von 820-840 nm;
einen Infrarot-Bildsensor, der dazu konfiguriert ist, vom Endoskop gesammeltes Licht zu empfangen; und
ein Bildverarbeitungsmodul, das dazu konfiguriert ist, ein Videosignal vom Infrarot-Bildsensor zu empfangen und ein Bild mit kohärentem Licht basierend auf dem empfangenen Videosignal zu bestimmen,
vorzugsweise eine RGB/IR-Kamera oder einen Strahlteiler zum Aufteilen des vom Ziel reflektierten Lichts in einen IR-Strahl und einen Weißlichtstrahl und eine IR-Kamera.

20. Endoskopisches System, vorzugsweise ein laparoskopisches System, aufweisend die Kopplungsvorrichtung nach einem der Ansprüche 1-17.

## Revendications

1. Dispositif de couplage (140) pour coupler une lumière cohérente et une lumière incohérente dans un premier guide de lumière (124) d'un système de distribution de lumière d'un système endoscopique (100_{1,2}), le système endoscopique (100_{1,2}) comprenant un endoscope (106) et une source de lumière incohérente (108) pour générer la lumière incohérente, par exemple, de la lumière blanche, le premier guide de lumière (124) étant configuré pour connecter le dispositif de couplage (140) à l'endoscope (106), le dispositif de couplage (140) comprenant :
une entrée optique (142) comprenant un connecteur d'entrée optique pour connecter de manière amovible le dispositif de couplage (140) à la source de lumière incohérente (108) via un second guide de lumière (158), l'entrée optique (142) étant configurée pour recevoir la lumière incohérente du système endoscopique (100_{1,2}) ;
une première source de lumière (146) pour générer une lumière cohérente d'une première longueur d'onde ;
une sortie optique (144) comprenant un connecteur de sortie optique pour connecter de manière amovible le dispositif de couplage (140) à l'endoscope (106) via le premier guide de lumière (124), la sortie optique (144) étant configurée pour fournir la lumière incohérente et la lumière cohérente de la première longueur d'onde au premier guide de lumière (124) ;
un commutateur optique (148) pour injecter alternativement la lumière incohérente et la lumière cohérente de la première longueur d'onde dans le premier guide de lumière (124), dans lequel la lumière cohérente et la lumière incohérente ont un trajet optique partagé au niveau de la sortie optique (144) ; et
un commutateur de commande pour générer un signal de déclenchement ou une entrée de déclenchement pour recevoir un signal de déclenchement, dans lequel le commutateur optique est configuré pour passer d'un premier état à un second état en réponse à la réception d'un signal de déclenchement ;
dans lequel dans au moins le premier état du dispositif de couplage (140), le dispositif de couplage (140) comprend un trajet de faisceau de lumière incohérente non obstrué pour propager la lumière incohérente de l'entrée optique (142) à la sortie optique (144), et la lumière cohérente n'est pas injectée dans le premier guide de lumière (124) ; et
dans lequel dans au moins le second état du dispositif de couplage (140), le dispositif de couplage (140) comprend un trajet de faisceau de lumière cohérente non obstrué pour propager la lumière cohérente de la source de lumière cohérente (146) à la sortie optique (144) et la lumière cohérente est injectée dans le premier guide de lumière (124).

2. Dispositif de couplage selon la revendication 1, dans lequel dans le premier état, le commutateur optique est configuré pour injecter la lumière incohérente dans le premier guide de lumière, et dans lequel dans le second état, le commutateur optique est configuré pour ne pas injecter la lumière incohérente dans le premier guide de lumière.

3. Dispositif de couplage selon la revendication 1 ou 2, dans lequel le commutateur optique comprend :
un premier miroir mobile entre une première position dans laquelle une surface réfléchissante du premier miroir est positionnée pour réfléchir une lumière incohérente se propageant sur le trajet de faisceau de lumière incohérente, et une seconde position dans laquelle la surface réfléchissante du premier miroir est positionnée pour réfléchir la lumière incohérente dans une direction éloignée du trajet de faisceau de lumière incohérente ;
et/ou un second miroir mobile entre une première position dans laquelle une surface réfléchissante du second miroir est positionnée pour réfléchir une lumière cohérente se propageant sur le trajet de faisceau de lumière cohérente, et une seconde position dans laquelle la surface réfléchissante du premier miroir est positionnée pour réfléchir la lumière cohérente dans une direction éloignée du trajet de faisceau de lumière cohérente.

4. Dispositif de couplage selon la revendication 3, dans lequel le commutateur optique est un commutateur à commande électrique avec un moteur, facultativement un servomoteur ; le moteur étant configuré pour déplacer le premier miroir et, facultativement, le second miroir, entre la première position et la seconde position.

5. Dispositif de couplage selon la revendication 1 ou 2, dans lequel le commutateur optique comprend :
un premier miroir commutable, commutable entre un état transparent et un état réfléchissant, positionné pour réfléchir, dans l'état réfléchissant, une lumière incohérente se propageant sur le trajet de faisceau de lumière incohérente ; et/ou un second miroir commutable, commutable entre un état transparent et un état réfléchissant, positionné pour réfléchir, dans l'état réfléchissant, une lumière cohérente se propageant sur le trajet de faisceau de lumière cohérente ;
ou dans lequel le commutateur optique comprend :
un premier bloqueur de faisceau lumineux configuré pour bloquer la lumière cohérente dans le premier état, de préférence le premier bloqueur de faisceau comprenant un obturateur mécanique, de préférence un obturateur rotatif, plus préférentiellement un obturateur rotatif motorisé, ou un bloqueur de faisceau optique, de préférence l'un parmi :
une valve de lumière à cristaux liquides, un modulateur acousto-optique, un modulateur électro-optique ou un modulateur photoélastique ; et, facultativement, un second bloqueur de faisceau lumineux configuré pour bloquer la lumière incohérente dans le second état, de préférence le second bloqueur de faisceau comprenant un obturateur mécanique, de préférence un obturateur rotatif, plus préférentiellement un obturateur rotatif motorisé, ou un bloqueur de faisceau optique, de préférence une valve de lumière à cristaux liquides.

6. Dispositif de couplage selon l'une quelconque des revendications précédentes, dans lequel le commutateur optique comprend :
une première lentille convergente pour réduire la divergence de la lumière incohérente à partir de l'entrée optique et une seconde lentille convergente pour focaliser la lumière incohérente dans le premier guide de lumière ; et
un miroir, positionné entre la première lentille et la seconde lentille, configuré pour diriger le faisceau de lumière cohérente et/ou le faisceau de lumière incohérente dans le premier guide de lumière.

7. Dispositif de couplage selon l'une quelconque des revendications précédentes, dans lequel le commutateur optique comprend :
un tube de lumière conique ayant une extrémité large pour recevoir la lumière incohérente de l'entrée optique et une extrémité étroite pour fournir la lumière incohérente à la sortie optique, le tube de lumière conique étant divisé en une première partie et une seconde partie par un plan intersectant le tube de lumière conique entre l'extrémité large et l'extrémité étroite, une normale du plan définissant un angle non nul avec un axe longitudinal du tube de lumière conique, de préférence, le tube de lumière conique comprenant en outre une saillie ayant une surface normale au trajet de lumière cohérente pour recevoir la lumière cohérente ; et
un miroir, positionné sur le plan entre la première partie et la seconde partie du tube de lumière conique sur l'axe longitudinal du tube de lumière conique, configuré pour diriger le faisceau laser dans le premier guide de lumière.

8. Dispositif de couplage selon la revendication 1 ou 2, dans lequel le commutateur optique comprend :
un guide de lumière interne pour guider la lumière incohérente d'une première extrémité du guide de lumière interne à une seconde extrémité du guide de lumière interne ; et
un corps de commutation comprenant au moins la seconde extrémité du guide de lumière interne, le corps de commutation étant mobile entre une première position correspondant au premier état et une seconde position correspondant au second état,
- première position dans laquelle le corps de commutation est configuré pour bloquer le trajet de faisceau de lumière cohérente, pour positionner la première extrémité du guide de lumière interne pour recevoir une lumière incohérente de l'entrée optique, et pour positionner la seconde extrémité pour injecter la lumière incohérente dans le premier guide de lumière ; et
- seconde position dans laquelle le corps de commutation est configuré pour ne pas bloquer le trajet de faisceau de lumière cohérente, et pour positionner au moins la seconde extrémité du guide de lumière interne de sorte que la lumière incohérente ne soit pas injectée dans le premier guide de lumière.

9. Dispositif de couplage selon la revendication 8, dans lequel le guide de lumière interne est un guide de lumière flexible, de préférence un faisceau de fibres réunies par fusion ou un guide de lumière liquide, et dans lequel la première extrémité du guide de lumière interne est connectée à l'entrée optique.

10. Dispositif de couplage selon la revendication 8 ou 9, dans lequel le commutateur optique est un commutateur à commande électrique avec un moteur, facultativement un servomoteur ; le moteur étant configuré pour déplacer le corps de commutation entre la première position et la seconde position via une came ou une manivelle.

11. Dispositif de couplage selon l'une quelconque des revendications précédentes, dans lequel le second guide de lumière est un guide de lumière flexible, de préférence un faisceau de fibres, un faisceau de fibres réunies par fusion ou un guide de lumière liquide.

12. Dispositif de couplage selon l'une quelconque des revendications précédentes, dans lequel la première source de lumière est un laser à bande étroite, de préférence ayant une bande passante spectrale inférieure à 1 nm, plus préférentiellement inférieure à 0,2 nm, encore plus préférentiellement inférieure à 0,1 nm.

13. Dispositif de couplage selon l'une quelconque des revendications précédentes, dans lequel la première source de lumière est un laser avec une puissance de sortie d'au moins 20 mW, de préférence d'au moins 100 mW, encore plus préférentiellement d'au moins 150 mW.

14. Dispositif de couplage selon l'une quelconque des revendications précédentes, dans lequel la première longueur d'onde est dans la partie rouge du spectre électromagnétique, de préférence dans la plage 600-700 nm, plus préférentiellement dans la plage 630-660 nm, ou dans lequel la première longueur d'onde est dans la partie infrarouge du spectre électromagnétique, de préférence dans la plage 700-1200 nm, plus préférentiellement dans la plage 700-900 nm, encore plus préférentiellement dans la plage 770-790 nm ou dans la plage 820-840 nm.

15. Dispositif de couplage selon l'une quelconque des revendications précédentes, dans lequel le système endoscopique comprend une caméra, de préférence une caméra RVB, la caméra étant configurée pour fournir un signal vidéo, et dans lequel le dispositif de couplage comprend en outre :
un connecteur d'entrée de signal vidéo pour recevoir le signal vidéo ;
un connecteur de sortie de signal vidéo pour fournir une sortie vidéo ; et
un module de traitement d'image pour générer des images de lumière cohérente, de préférence des images de fluorescence et/ou des images de contraste de spectre laser sur la base du signal vidéo lorsque le commutateur optique injecte de la lumière cohérente dans le premier guide de lumière.

16. Dispositif de couplage selon la revendication 15, dans lequel
dans le premier état, le signal vidéo est bouclé vers la sortie vidéo ; et
dans lequel dans le second état, le signal vidéo est fourni au module de traitement d'image, le module de traitement d'image étant configuré pour déterminer une ou plusieurs images de sortie, de préférence des images de contraste de granularité laser ou des images de fluorescence, sur la base du signal vidéo, et les une ou plusieurs images de sortie étant fournies au niveau du connecteur de sortie de signal vidéo.

17. Dispositif de couplage selon l'une quelconque des revendications précédentes, comprenant en outre un premier capteur, de préférence un capteur optique, et un dispositif de commande, connecté au premier capteur, dans lequel :
le premier capteur est configuré pour envoyer un premier signal au dispositif de commande uniquement si le premier guide de lumière est connecté au connecteur de sortie optique ; et
le dispositif de commande est configuré pour éteindre ou bloquer la première source de lumière cohérente, si le dispositif de commande ne reçoit pas le premier signal.

18. Système de traitement d'image pour générer des images de lumière cohérente avec un système endoscopique, le système endoscopique comprenant une source de lumière incohérente, une unité de traitement vidéo et un endoscope, l'endoscope étant connecté à une caméra, le système de traitement d'image comprenant un dispositif de couplage selon l'une quelconque des revendications 1 à 12 et un dispositif de traitement vidéo, le dispositif de traitement vidéo comprenant :
- un connecteur d'entrée de signal vidéo pour recevoir un signal vidéo de la caméra ;
- un premier connecteur de sortie de signal vidéo pour fournir une sortie vidéo, le premier connecteur de sortie de signal vidéo étant configuré pour être connecté à une entrée de signal vidéo de l'unité de traitement vidéo du système endoscopique ; et
- un module de traitement d'image pour générer des images de lumière cohérente sur la base du signal vidéo lorsque le commutateur optique est dans le premier état.

19. Système d'imagerie pour générer une image de lumière cohérente infrarouge avec un système endoscopique, le système endoscopique comprenant une source de lumière incohérente, un endoscope et un système de distribution de lumière pour distribuer de la lumière à l'endoscope, le système d'imagerie comprenant :
un dispositif de couplage selon l'une quelconque des revendications 1 à 11, la première longueur d'onde étant sélectionnée dans la partie infrarouge du système électromagnétique, de préférence dans la plage 700-1200 nm, plus préférentiellement dans la plage 700-900 nm, encore plus préférentiellement dans la plage 770-790 nm ou dans la plage 820-840 nm ;
un capteur d'imagerie infrarouge, configuré pour recevoir la lumière collectée par l'endoscope ; et
un module de traitement d'image, configuré pour recevoir un signal vidéo du capteur d'imagerie infrarouge et pour déterminer une image lumineuse cohérente sur la base du signal vidéo reçu,
de préférence une caméra RVB/IR ou un séparateur de faisceau pour diviser la lumière réfléchie par la cible en un faisceau IR et un faisceau de lumière blanche et une caméra IR.

20. Système endoscopique, de préférence système laparoscopique, comprenant le dispositif de couplage selon l'une quelconque des revendications 1 à 17.
